(19) Europäisches Patentamt
European Patent Office
Office européen des brevets

(11) Publication number: **0 466 944 A1**

# (12) EUROPEAN PATENT APPLICATION published in accordance with Art. 158(3) EPC

(21) Application number: **91904621.9**

(22) Date of filing: **15.02.91**

(86) International application number:
**PCT/JP91/00183**

(87) International publication number:
**WO 91/12245 (22.08.91 91/19)**

(51) Int. Cl.5: **C07D 265/24**, C07D 413/04, C07K 5/06, C07K 5/08, A61K 31/535, A61K 37/64

(30) Priority: **15.02.90 JP 32440/90**

(43) Date of publication of application:
**22.01.92 Bulletin 92/04**

(84) Designated Contracting States:
**AT BE CH DE DK ES FR GB IT LI NL SE**

(71) Applicant: **TEIJIN LIMITED**
**6-7, Minamihonmachi 1-chome Chuo-ku**
**Osaka-shi Osaka 541(JP)**

(72) Inventor: **OSHIDA, Junichi**
**1-17-17-301, Shinmei**
**Hino-shi, Tokyo 191(JP)**
Inventor: **KAWABATA, Hiroshi**
**3-2-5-102, Tamadaira**
**Hino-shi, Tokyo 191(JP)**
Inventor: **KATO, Yoshinori**
**3-24-1, Tamadaira**
**Hino-shi, Tokyo 191(JP)**
Inventor: **KOKUBO, Masayuki**
**2-16-6-103, Hinohonmachi**
**Hino-shi, Tokyo 191(JP)**
Inventor: **UEJIMA, Yasuhide**
**210, Heim SI, 1-6-14, Shinmei**
**Hino-shi, Tokyo 191(JP)**
Inventor: **SATO, Osami**
**A-302, Kyoritsu-Reliance-Toyoda**
**2-33-12, Asahigaoka, Hino-shi, Tokyo 191(JP)**
Inventor: **FUJII, Katsuhiko**
**3-2-18, Daimachi**
**Hachioji-shi, Tokyo 193(JP)**

(74) Representative: **Votier, Sidney David et al**
**CARPMAELS & RANSFORD 43, Bloomsbury Square**
**London WC1A 2RA(GB)**

## (54) 4H-3,1-BENZOXAZIN-4-ONE COMPOUND AND ELASTASE INHIBITOR COMPOSITION CONTAINING THE SAME.

(57) A 4H-3,1-benzoxazin-4-one compound of general formula (I), salts thereof, and pharmaceutical composition containing the same as the active ingredient. They have a protease inhibiting effect, particularly a selective inhibiting effect on a human leukocyte elastase and are excellent in water solubility and residence in the tissue.

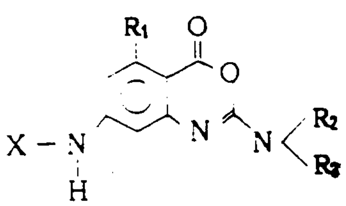

[ I ]

EP 0 466 944 A1

Field of the Art:

The present invention relates to 4H-3,1-benzoxazin-4-one compounds and the pharmaceutical composition containing the same as an active ingredient.

Particularly, the present invention relates to 4H-3,1-benzoxazin-4-one compounds which have the inhibitory effect against proteases, particularly the selective inhibitory effect against elastase, for prophylaxis and treatment of diseases caused by the action of protease on proteins such as mammalian elastin, especially, preventing injury of tissues, and a variety of inflammations and degenerations, which are caused by the lytic effects of elastase, and relates to pharmaceutical compositions which contain these compounds as an active ingredient, and can be used in prophylaxis and treatment for diseases caused by serine protease, especially for prophylaxis and treatment for diseases caused by human leukocyte elastase.

Background of the Art:

Elastin is a fibrous protein which constitutes the major components of the elastic fibers in connective tissues with rubber-like elasticity, and largely distributes in lungs, bronchi, and aortas. Elastase is a group of proteases which can hydrolyze elastin and produced by mammalian pancreas, polymorphonuclear leukocytes or microorganisms. The leukocyte elastase has an important action as a digestive enzyme to decompose phagocyted bacteria, but, when leaked out of the leukocytes, the elastase degradates tissue elastin, causing injuries, a variety of inflammations and degenerations of tissues. Such excessive digestion of elastin by the elastase has been considered to be a cause of pulmonary emphysema, adult respiratory distress syndrome, pulmonary fibrosis, bronchitis, pneumonia, rheumatoid arthritis, arteriosclerosis, sepsis, shock, pancreatitis, nephritis, and certain kinds of dermatosis. Consequently, an elastase inhibitor can be thought to be useful as a therapeutic agent or preventive against these diseases.

As a compound having the elastase inhibitory activity, have been known 4H-3,1-benzoxazin-4-one compounds having the basic structure of the following formula:

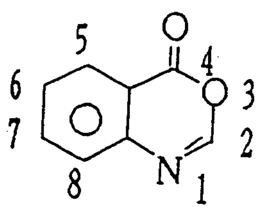

For example, Teshima et al., J. Biol. Chem. 257, 5085 - 5091 (1982) described a variety of 2-alkyl-4H-3,1-benzoxazin-4-one, while Hedstrom et al., Biochemistry, 23, 1753 - 1759 (1984) disclosed 2-ethoxy-4H-3,1-benzoxazin-4-one. Spencer et al., Biochem. Biophys. Res. Commun., 140, 928 - 933 (1986) also reported that 5-methyl-substituted 2-alkyl-4H-3,1-benzoxazin-4-one had excellent elastase inhibitory activity. Further, Stein et al., Biochemistry, 26, 4126 - 4130 (1987) and Radhakrishnan et al., J. Mol. Biol., 198, 417 - 424 (1987) reported the mechanism of elastase inhibition by benzoxazinone. Additionally, Japanese Patent Specification Laid-open No. 60-169469 (1985) disclosed 2-amino derivatives of 4H-3,1-benzoxazin-4-ones, and Japanese Patent Specification Laid-open No. 62-30770 (1987) showed 2-oxy derivatives of 4H-3,1-benzoxazin-4-ones.

It has been known that most of these compounds reveal stronger inhibitory activity against chymotrypsin than against elastase.

2,7-Diamino derivatives of 4H-3,1-benzoxazin-4-ones were also proposed by the present inventors, as 4H-3,1-benzoxazin-4-one derivatives having stronger inhibitory activity against elastase than against chymotrypsin (W088/09790).

In spite of strong inhibitory activity and high selectivity against elastate, the 4H-3,1-benzoxazin-4-one derivatives already disclosed by the present inventors have become clear that these compounds have a room for improvement such as low solubility in water and rapid diappearance from the pulmonary tissues in a short time after administered into mammalian respiratory tracts.

Disclosure of the invention:

The object of the present invention is to provide novel 4H-3,1-benzoxazin-4-ones having strong

inhibitory action against serine proteases and pharmaceutical compositions containing the same as an active ingredient.

Another object of the invention is to provide novel 4H-3,1-benzoxazin-4-ones having selective and strong inhibitory activity against particularly human leukocyte elastase among serine proteases and pharmaceutical compositions containing the same as an active ingredient.

Further, another object of the invention is to provide novel 4H-3,1-benzoxazin-4-ones which have strong inhibitory activity and high selectivity against human leukocyte elastase with excellent solubility in water and high retention in lung tissues and pharmaceutical compositions containing the same as an active ingredient.

The objects stated above are attained by the compounds of the present invention. In other words, the invention is 4H-3,1-benzoxazin-4-one compounds of the formula [I]

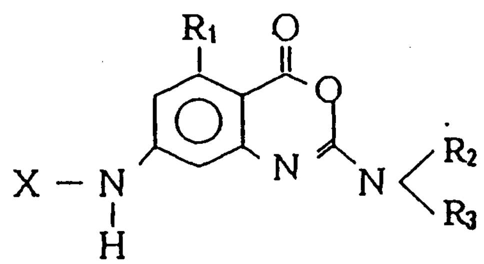

[ I ]

wherein

(i) X is a substituent represented by formula [A]

$Y_1$-$A_1$-     [A]

or formula [B]

$Y_2$-($A_2$)m-$A_3$-     [B]

$A_1$ is an amino acid residue or a peptide which is constituted with 2 or 3 amino acid residues (the side chains of the amino acid residues may be protected),

$A_2$ represents the residue of an amino acid selected from glycine, alanine, valine and leucine or a dipeptide constituted from these amino acids, $A_3$ represents the residue of an amino acid selected from lysine, glutamic acid and aspartic acid (the side chains of the amino acid residues may be protected),

$Y_1$ represents a protecting group for an amino group,

$Y_2$ represents a hydrogen atom or sulfonyl group (wherein when the side chain of the amino acid residue in $A_3$ is protected, it represents a hydrogen atom),

m represents 0 or 1;

(ii) $R_1$ represents a hydrogen atom or a lower alkyl;

and

(iii) as to $R_2$ and $R_3$,

(a) when X represents formula [A], $R_2$ represents a lower alkyl bearing 1 or 2 carboxyl groups, and $R_3$ represents a hydrogen atom, a lower alkyl, or a lower alkyl bearing 1 or 2 carboxyl groups, or they bond to each other to form a 5, 6 or 7-membered ring substituted with 1 or 2 carboxyl groups or lower alkyl groups bearing 1 or 2 carboxyl groups, or

(b) when X represents formula [B], $R_2$ represents a lower alkyl group and $R_3$ represents a hydrogen atom,

their salts and their pharmaceutical compositions.

The pharmaceutical compositions of the present invention contains 4H-3,1-benzoxazin-4-one of formula [I] or a mixture thereof in its medicinally effective amount with medicinally permissible carriers.

The best embodiment for the present invention:

In formula [I], X shows a substituent represented by formula [A]

$Y_1$-$A_1$-     [A]

or formula [B]

$Y_2$-$(A_2)$m-$A_3$-     [B]

wherein

$A_1$ represents the residue of an amino acid or a peptide which is constituted with the residues of 2 or 3 amino acids (the side chains of these amino acid residues may be protected).

The amino acid residue represented by $A_1$ or the amino acid residues constituting peptides include the residues of D, L-optical isomers or racemic isomers of $\alpha$ -, $\beta$ - and $\gamma$ -amino carboxylic acids, and such amino acids are preferably selected from, for example, D- and L-optical isomers or racemates of alanine, asparagine, aspartic acid, cysteine, cystine, glutamic acid, glutamine, glycine, histidine, isoleucine, leucine, lysine, methionine, phenylalanine, proline, serine, threonine, tryptophan, tyrosine, valine, homoserine, homocysteine, hydroxyproline, ornithine, thyroxine, norvaline, norleucine, phenylglycine, $\beta$ -alanine, and $\gamma$ -aminobutyric acid.

More preferred amino acids are L-alanine, glycine, L-isoleucine, L-leucine, L-phenylalanine, L-proline, L-valine, L-norvaline, L-norleucine, L-phenylglycine, L-lysine bearing the $\epsilon$ -amino group protected with a carbobenzoxy group, L-aspartic acid bearing the $\beta$ -carboxyl group protected in the form of a benzyl ester, L-glutamic acid bearing the carboxyl group protected in the form of $\gamma$ -benzyl ester or the like.

In formula [A], the side chains of the amino acid residues represented by $A_1$ may be protected with a protecting group. As such a protecting group for protecting the side chains (amino, carboxyl, guanidino, imidazolyl, mercapto or hydroxyl groups) of amino acid residues represented by $A_1$, are cited, for example, carbobenzoxy, succinyl, methoxysuccinyl, acetyl, trifluoroacetyl, tert-butoxycarbonyl, isonicotinyloxycarbonyl, tosyl groups or the like for the amino group. Further, as a group for protecting the carboxyl group, are known benzyl ester, 4-picolyl ester or the like; for protecting guanidino and imidazolyl groups, carbobenzoxy and tosyl groups; for mercapto group, S-benzyl group; and for hydroxyl group, O-benzyl. These protecting groups which are useful for the present invention, however, are not limited to those cited above.

In formula [A], $Y_1$ represents a protecting group for amino group and the above-stated amino group-protecting groups can be cited as such a protecting group. The protecting group is preferably selected from carbobenzoxy, tert-butoxycarbonyl, methoxysuccinyl and acetyl groups among them.

In the cases where X is represented by [B] in formula [I], $A_2$ represents the residue of amino acids selected from glycine, alanine, valine and leucine or dipeptides constituted with these amino acids, while $A_3$ represents the residue of amino acids selected from lysine, glutamic acid and aspartic acid (the side chains of these amino acid residues may be protected). m represents 0 or 1.

These amino acid residues includes the residues of their D-, L-optical and racemic isomers, as in the amino acid residues of the above-stated $A_1$.

The residue of an amino acid selected from glycine, alanine and valine is preferred as $A_2$ among them.

As $A_3$, is preferred the residue of lysine or glutamic acid among them.

When the side chain of the amino acid residue is protected in $A_3$, the similar groups cited in the protecting groups stated in $A_1$ can be cited as such a protecting group.

In formula [B], $Y_2$ represents a hydrogen atom or a sulfonyl group (wherein it represents a hydrogen atom, when the side chain of the amino acid residue of $A_3$ is protected).

As a sulfonyl group, can be cited substituted or unsubstituted aromatic sulfonyl groups. Such substituents are one or more than two identical or different halogen atoms (fluorine atoms, chlorine atoms, bromine atoms, or the like) and lower alkoxy groups of one to six carbon atoms (methoxy, ethoxy, isopropoxy or the like).

Chlorine atoms, methyl groups, methoxy groups are preferred as these substituents among them. The aromatic sulfonyl group is, for example, benzenesulfonyl, $\alpha$ -naphthylsulfonyl and $\beta$ -naphthylsulfonyl and benzenesulfonyl is preferably cited.

In formula [I], $R_1$ is a hydrogen atom or a lower alkyl and the alkyl group has preferably one to six carbon atoms. Such an alkyl group is preferably selected from methyl, ethyl, propyl, butyl, pentyl, hexyl and their isomers.

In formula [I], generally $R_1$ preferably represents a hydrogen atom, methyl or ethyl group.

As to $R_2$ and $R_3$ in formula [I], $R_2$ represents a lower alkyl having 1 or 2 carboxyl groups and $R_3$ is a hydrogen atom, a lower alkyl or a lower alkyl having 1 or 2 carboxyl groups, when X represents formula [A], or $R_2$ and $R_3$ bond to each other to represent a 5, 6 or 7-membered ring substituted with 1 or 2 carboxyl groups or lower alkyls having 1 or 2 carboxyl groups.

The lower alkyl group having 1 or 2 carboxyl groups, which is represented by $R_2$ in formula [I], is

preferably of 1 to 6 carbon atoms, for example, carboxymethyl, 1-carboxyethyl, 3-carboxypropyl, 1,2-dicarboxyethyl, 1,3-dicarboxypropyl and the like.

In formula [I], the lower alkyl represented by $R_3$ is preferably of 1 to 6 carbon atoms and selected from methyl, ethyl, propyl, butyl, pentyl, hexyl and their isomeric forms.

In formula [I], the lower alkyl having 1 or 2 carboxyl groups represented by $R_3$ is preferably of 1 to 6 carbon atoms and selected from, for example, carboxymethyl, 1-carboxyethyl, 3-carboxypropyl, 1,2-dicarboxyethyl or 1,3-dicarboxypropyl.

In general, $R_3$ in formula [I] preferably represents a hydrogen atom, methyl, ethyl or carboxymethyl group.

In formula [I], $R_2$ and $R_3$ can bond to each other to form, together with a nitrogen atom to which $R_2$ and $R_3$ bond, a 5, 6 or 7-membered ring substituted with 1 or 2 carboxyls or lower alkyls having 1 or 2 carboxyl groups, for example, 2-carboxypyrrolidine, 3-carboxypyrrolidine or 4-carboxypiperidine, or carboxy-lower-alkylpyrrolidine or carboxy-lower-alkyl-piperidine.

In formula [I], when X represents formula [B], $R_2$ represents a lower alkyl, while $R_3$ represents a hydrogen atom. When $R_2$ represents a lower alkyl, such alkyl group is defined as stated above.

As salts of the compounds of formula [I], the salts formed from inorganic and organic bases and the carboxyl groups included in $R_2$ or $R_3$, or from the carboxyl groups attaching to the side chains of amino acid residues or the amino acid residues in peptides, and inorganic or organic acid adducts which can be formed from amino, guanidino or imidazolyl groups are cited. As salts derived from inorganic base, ammonium, potassium, sodium, calcium, magnesium and the like are included. The salts derived from organic base are those of diethylamine, isopropylamine, ethanolamine, piperidine and the like. The acid adducts are formed using an inorganic acid such as hydrochloric acid, hydrobromic acid, sulfuric acid, nitric acid, phosphoric acid or the like or an organic acid such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, methanesulfonic acid, ethanesulfonic acid, p-toluenesulfonic acid, salicylic acid or the like.

When X represents formula [A] in formula [I]. 4H-3,1-benzoxazin-4-one can be obtained through the following synthetic route:

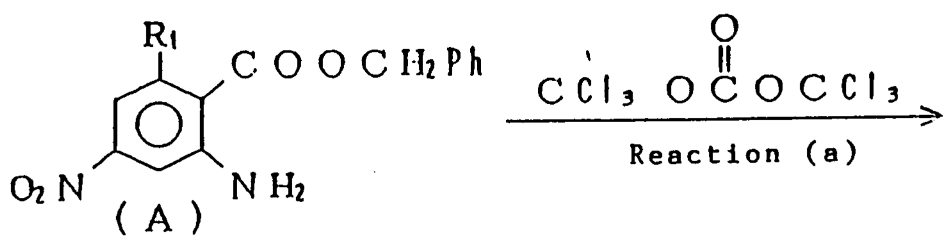


(A)

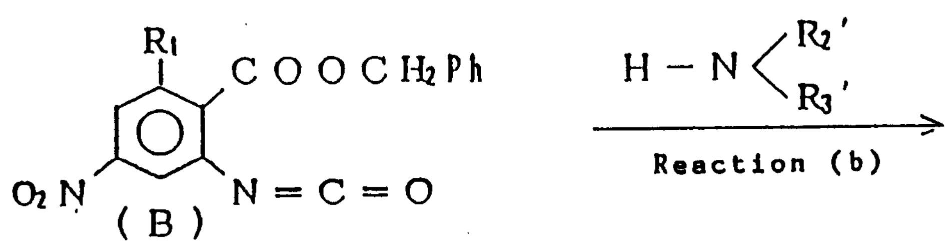


(B)

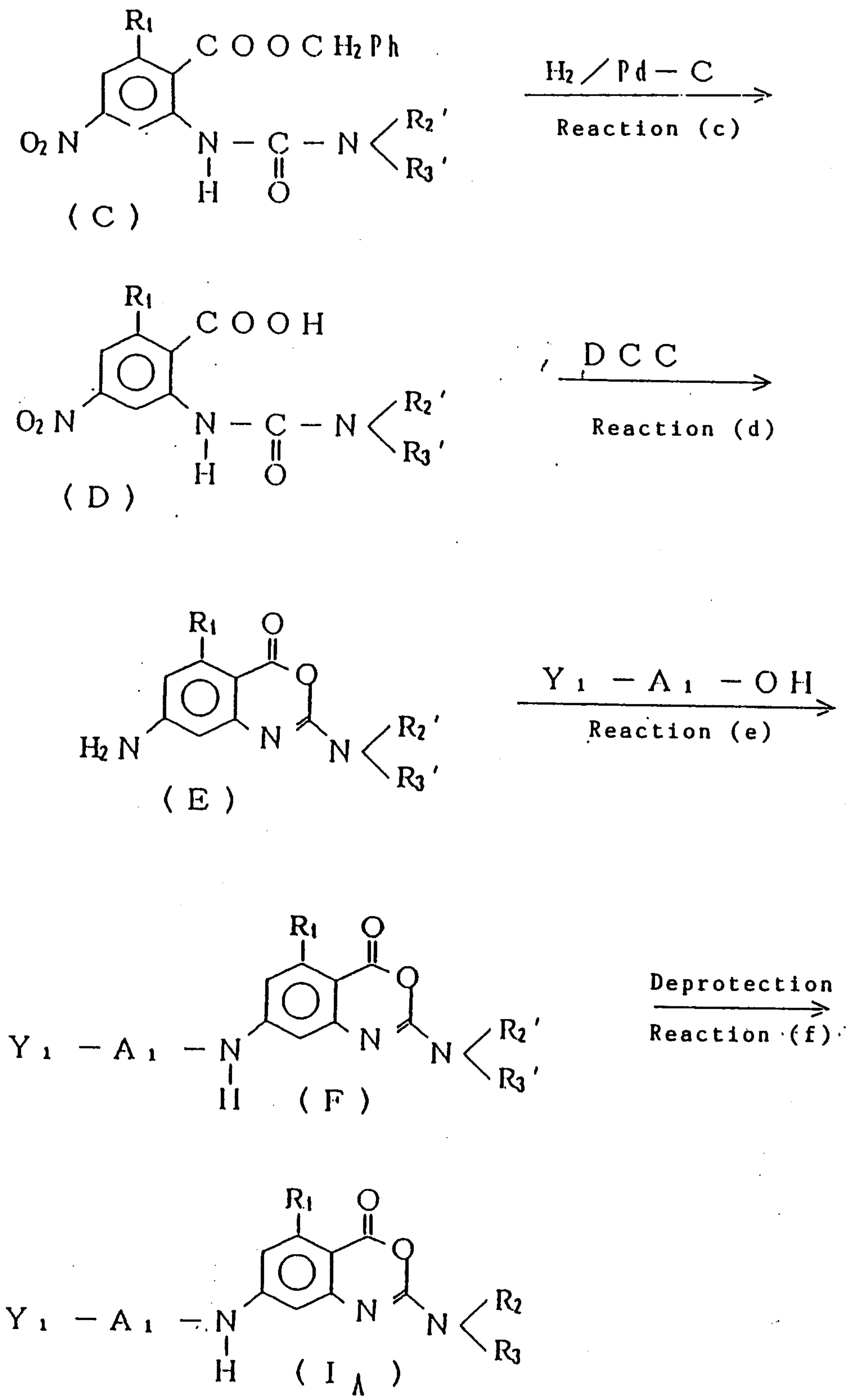


wherein

$R'_2$ and $R'_3$ are carboxyl-protected groups in $R_2$ and $R_3$, respectively.

In reaction (a), a benzyl substituted or unsubstituted 4-nitroanthranilate of formula (A) is allowed to react with bis(trichloromethyl) carbonate to form a phenyl isocyanate derivative of formula (B). The process is described by Eckert et al., Angew. Chem. Int. Ed. Engl., 26, 894 - 895 (1987).

In reaction (b), the phenyl isocyanate of formula (B) reacts an amine of formula [II]

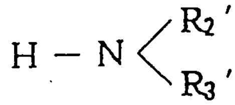

[ II ]

wherein

$R'_2$ and $R'_3$ are carboxyl-protected groups in $R_2$ and $R_3$, respectively.

to give benzyl 2-ureido-4-nitroanthranilate of formula (C). This reaction can be carried out by allowing both of the compounds to contact with each other in the presence of an inert organic solvent. Tetrahydrofuran, ether, benzene and the like are preferably organic solvents. The reaction is carried out at room temperature for 1 to 48 hours, preferably 3 to 20 hours.

In the amine of formula [II], $R'_2$ and $R'_3$ represent carboxyl-protected groups in $R_2$ and $R_3$, respectively. tert-Butyl group, trichloroethyl group or the like is used as a group for protecting carboxyls.

In reaction (c), benzyl 2-ureido-4-nitroanthranilate of formula (C) is converted into 2-ureido-4-aminoanthranilic acid derivative of formula (D) through a known process. The reaction (c) is preferably achieved by catalytic reduction with a hydrogen gas in the presence of a palladium-carbon catalyst.

In reaction (d), the 2-ureido-4-aminoanthranilic acid derivative of formula (D) is cyclized with a dehydrative condensing agent into 7-amino-4H-3,1-benzoxazin-4-one derivative of formula (E). Ethyl acetate, methylene chloride, benzene or the like is used as a preferable organic solvent. As a preferable dehydrative condensing agent, is cited N,N'-dicyclohexylcarbodiimide (DCC), 1-(3-dimethylaminopropyl)-3-ethylcarbodiimide (EDC) or the like. The reaction can be effected at room temperature for 1 to 48 hours, preferably for 3 to 20 hours.

In reaction (e), 7-amino-4H-3,1-benzoxazin-4-one derivative of formula (E) is subjected to condensation reaction with the carboxyl group of an amino acid or peptide bearing protected amino groups, which is represented by $Y_1$-$A_1$-OH to form 7-[$Y_1$-$A_1$]-amino-4H-3,1-benzoxazin-4-one derivative of formula (F). A variety of known processes for peptide bond formation can be applied to this condensation reaction (see N. Izumiya et al., The base and experiments for peptide syntheses (written in Japanese) (Marusen, Tokyo) 1985). In this condensation reaction (e), it is preferred that the carboxyl groups of an amino acid or peptide bearing protected amino groups are activated and subjected to condensation reaction with 7-amino-4H-3,1-benzoxazin-4-one derivative of formula (E) obtained in reaction (d). In the more preferable process, the amino acid or peptide having protected amino groups is allowed to react with a monoalkyl carbonate ester chloride to form a mixed acid anhydride, which is allowed to react with the compound of formula (E). The monoalkyl carbonate ester chloride to be preferably used in this process is isobutyl chloroformate (IBCF).

In reaction (f), the protecting groups are removed from the carboxyl groups included in $R'_2$ and $R'_3$, the substituents in the position 2 of 7-[$Y_1$-$A_1$]-amino-4H-3,1-benzoxazin-4-one derivative of formula (F) to give a 4H-3,1-benzoxazin-4-one compound of formula [$I_A$] of the present invention. This deprotecting reaction depends on the protecting group for the carboxyl groups included in $R'_2$ and $R'_3$, but, for example, the protecting groups can be readily removed with an acid, when tert-butyl is used for the protection.

In formula (A), the starting substance is commercially available in case that $R_1$ represents a hydrogen atom. Further, most of amino acids whose amino groups are protected with a carbobenzoxy or tert-butoxycarbonyl group, or which have protected side chains, are also commercially available. In addition, the amino acids bearing amino groups protected with methoxysuccinyl group can be prepared from an amino acid and monomethyl succinate (which is commercially available) by a known peptide bond formation reaction.

When $R_1$ represents an alkyl in the compound of formula [A] as a starting substance for the compound of formula [$I_A$], the compound of formula [A], namely benzyl 4-nitro-6-alkylanthranilate can be prepared by the following process:

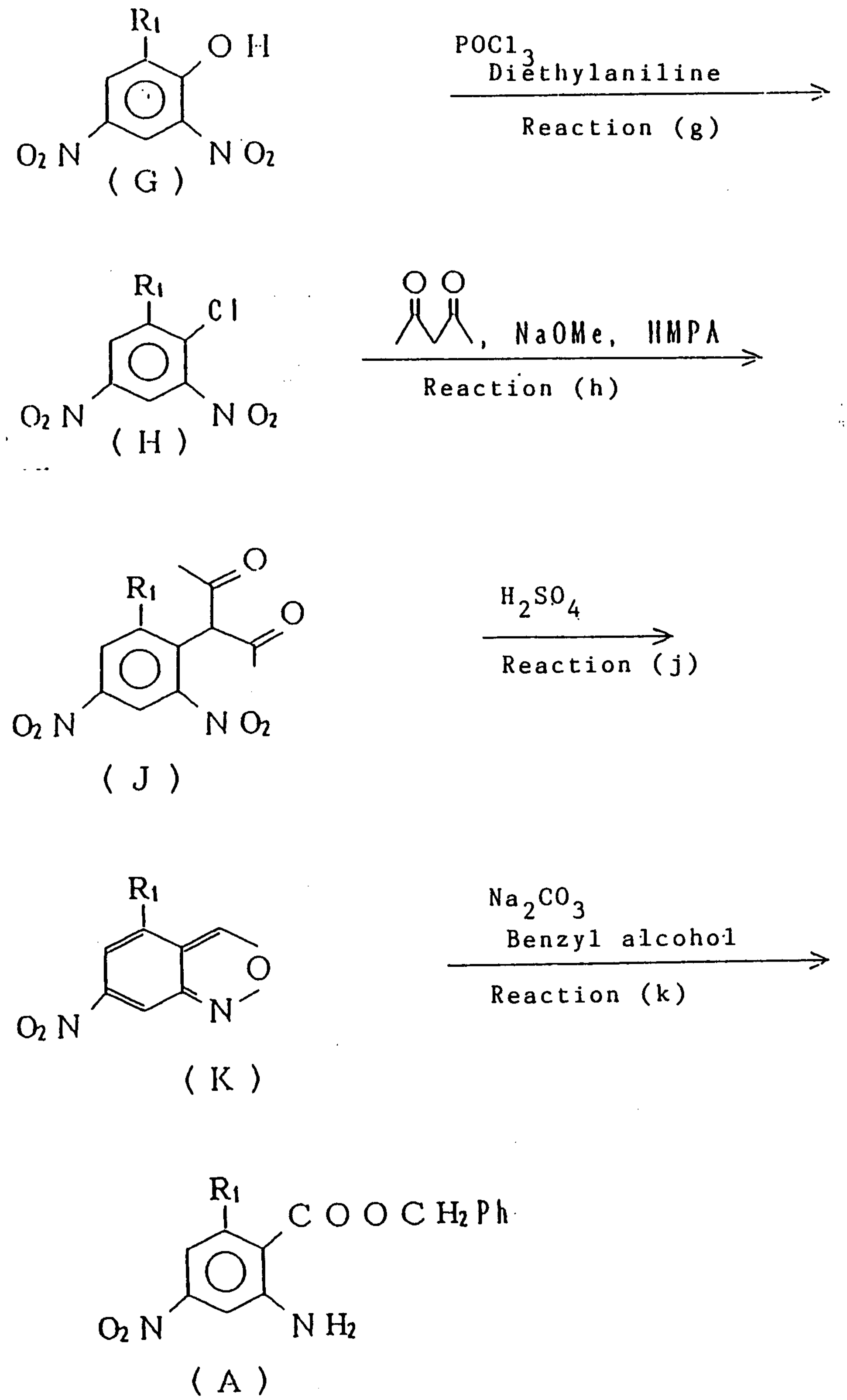


( G ) ( H ) ( J ) ( K ) ( A )

In reaction (g), the phenol derivative of formula (G) is converted into the corresponding chloro compound of formula (H) by the method described by Boothroyd et al., J. Chem. Soc., 1504 - 1508 (1953). In reaction (h), the product of formula (H) is allowed to react with pentane-2,4-dione and sodium methoxide according to Gambhir et al., J. Indian Chem. Soc., 41, 43 - 46 (1964) to form (2-alkyl-4,6-dinitrophenyl)-diacetylmethane of formula (J). The compound of formula (J) is cyclized with conc. sulfuric acid into the

compound of (K) in reaction (j). Finally in reaction (k), the product of formula (K) is heated in tetrahydrofuran in the presence of sodium carbonate and benzyl alcohol to give benzyl 4-nitro- 6-alkylanthranilate of formula (A).

The 4H-3,1-benzoxazin-4-one, namely the compound of formula [I] in case that X represents formula [B], can be synthesized through, for example, the following 3 routes:

(i) the case where $Y_2$ represents a hydrogen atom in formula [B]

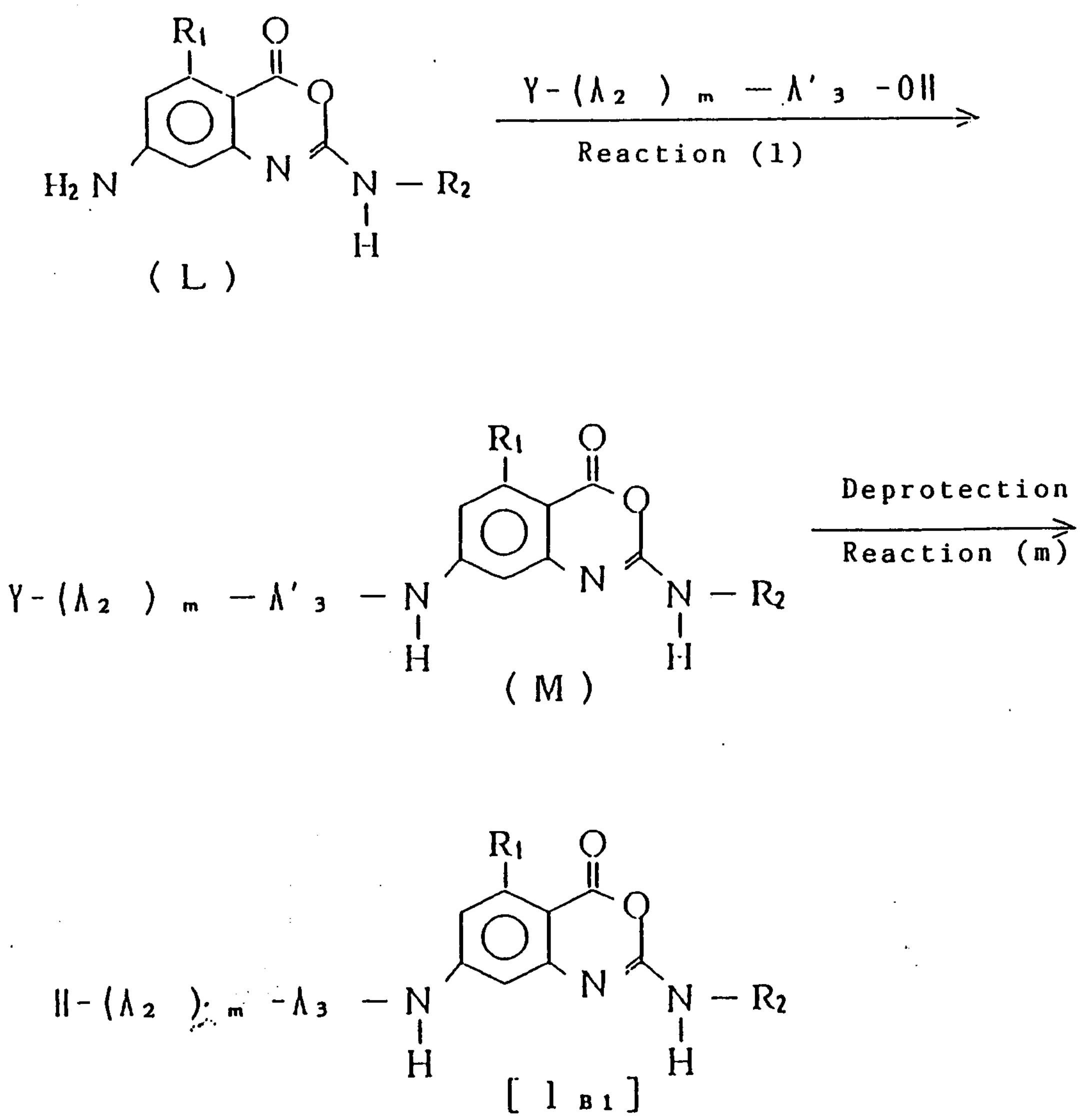


(L)

(M)

[I B1]

wherein

Y represents a protecting group for an amino group and

A'$_3$ represents an amino acid residue whose side chain is protected in $A_3$.

(ii) The case where $Y_2$ represents a sulfonyl group in formula [B]

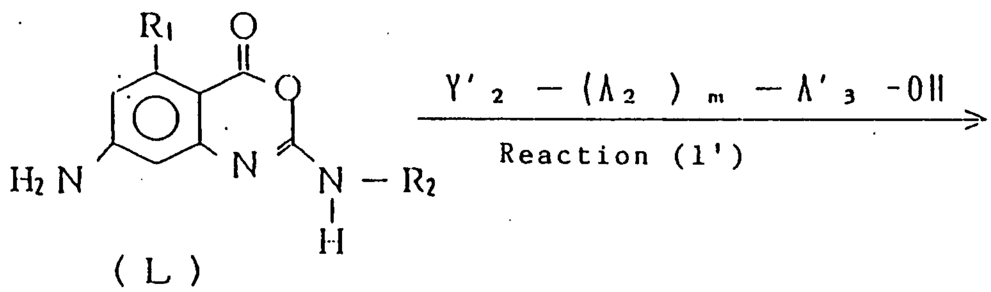


( L )

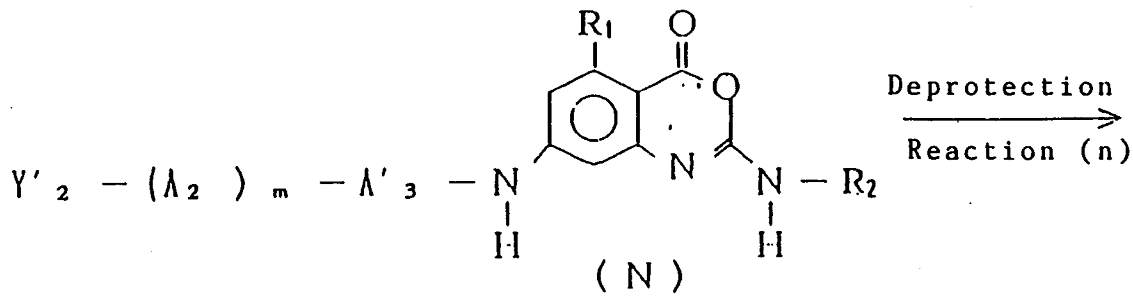


( N )

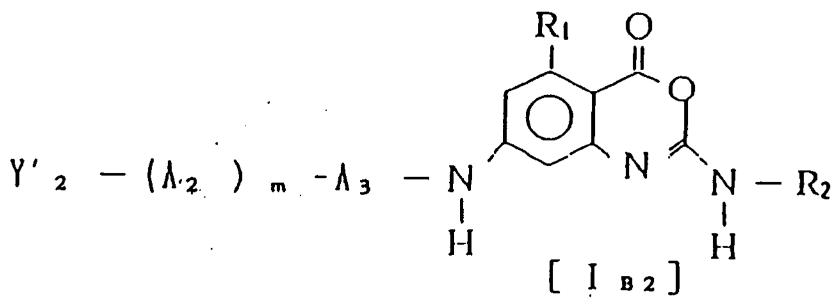

[ $I_{B2}$ ]

or

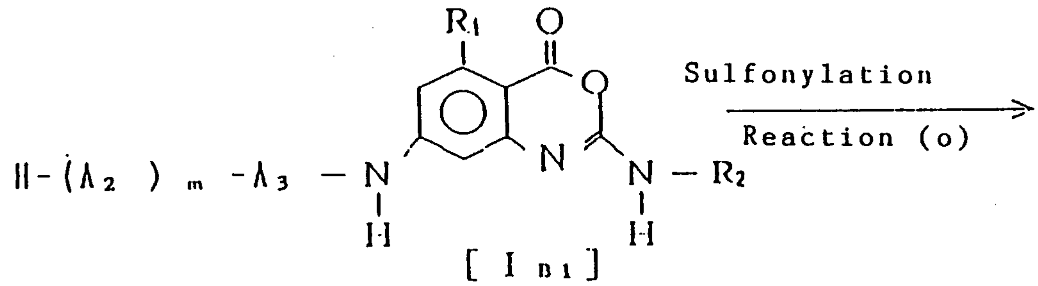


[$I_{B1}$]

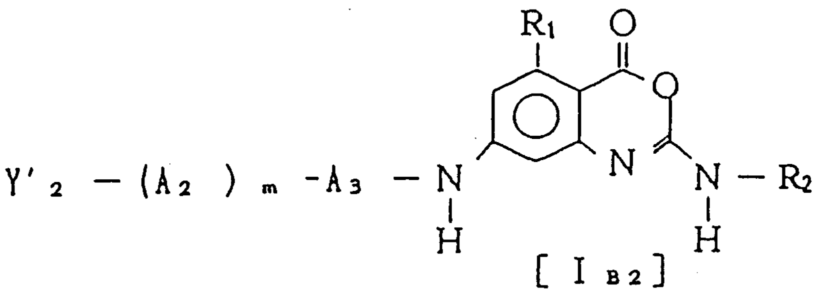

[$I_{B2}$]

wherein

$Y'_2$ represents a sulfonyl group, and

$A'_3$ represents an amino acid residue whose side chain is protected in $A_3$.

In reaction (1), a 7-amino-4H-3,1-benzoxazin-4-one derivative of formula (L) is subjected to condensation reaction with an amino acid or peptide represented by the following formula [III]:

$Y-(A_2)m-A'_3-OH$ [III]

wherein

Y represents an amino-protecting group, and $A'_3$ is an amino acid residue whose side chain is protected in $A_3$,

to form 7-[$Y-(A_2)m-A'_3$]-amino-4H-3,1-benzoxazin-4-one derivative of formula (M). This condensation reaction can be carried out in the same manner as in reaction (e).

In reaction (m), the protecting group Y is removed from 7-[$Y-(A_2)m-A'_3$]-amino-4H-3,1-benzoxazin-4-one derivative of formula [M] to give a 4H-3,1-benzoxazin-4-one compound of formula [$I_{B1}$]. This deprotection can be carried out in a similar manner to that in reaction (f). Further, in this process, the protecting group for the side chain of amino acid residue in $A'_3$ also may be removed simultaneously.

In reaction (1'), a 7-amino-4H-3,1-benzoxazin-4-one derivative of formula (L) is subjected to condensation reaction with an amino acid or peptide represented by the following formula [IV]:

$Y'_2-(A_2)m-A'_3-OH$ [IV]

wherein

$Y'_2$ represents a sulfonyl group, and

$A'_3$ is an amino acid residue whose side chain is protected in $A_3$,

to form 7-[$Y'_2-(A_2)m-A'_3$]-amino-4H-3,1-benzoxazin-4-one derivative of formula (N). This condensation reaction can be carried out in the same manner as in reaction (e).

In reaction (n), the side chain-protecting group in $A_3$ of 7-[$Y'_2-(A_2)m-A'_3$]-amino-4H-3,1-benzoxazin-4-one derivative of formula [N] is removed to give a 4H-3,1-benzoxazin-4-one compound of formula [$I_{B2}$]. This deprotection can be carried out in a similar manner to that in reaction (f).

Further, in reaction (o), 4H-3,1-benzoxazin-4-one of [$I_{B1}$] is sulfonylated to prepare a 4H-3,1-benzoxazin-4-one compound of formula [$I_{B2}$] of the invention. This sulfonylation reaction can be conducted by using a sulfonyl chloride such as p-chlorobenzenesulfonyl chloride, p-toluenesulfonyl chloride, or the like in an organic solvent. As an organic solvent, are preferably cited methylene chloride, tetrahydrofuran, benzene or

the like. Additionally, a base such as triethylamine or N-methylmorpholine is preferably added. The reaction is carried out at room temperature for 1 to 48 hours, preferably for 3 to 20 hours.

The compounds of formula (L) which is used as a starting substance for the compounds of formula [$I_{B2}$] of the present invention can be synthesized by the method which has been proposed by the present inventors (WO 88/09790).

In such a manner, the compounds of the present invention and the compounds obtained according to the present invention can be subjected to salt formation reaction with an inorganic acid or base or an organic acid or base to prepare their salts.

As concrete examples suitable for the compounds represented by formula [I] of the present invention, are cited those bearing substituents shown in the following table.

When asymmetric carbons are contained in the structure of the compounds, the present invention includes all of the optical isomers and all of the salts of these compounds.

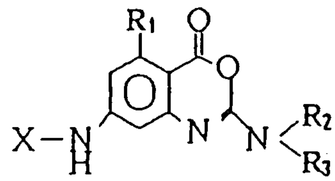

in the case where X is represented with formula [A] ($Y_1$-$A_1$-)

| Compound No. | $R_1$ | $R_2$ ( or $-N<^{R_2}_{R_3}$ ) | $R_3$ | $Y_1$ $-A_1$ $-$ |
|---|---|---|---|---|
| 101 | $CH_3$ | 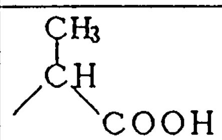 | H | Z−Phe − |
| 102 | $CH_3$ | 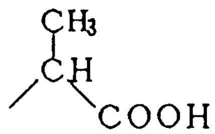 | H | Z−Ala − |
| 103 | $CH_3$ | 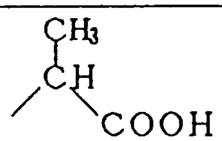 | H | Z−Val-Pro − |
| 104 | $CH_3$ | 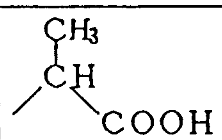 | $CH_3$ | Z−Leu-Phe − |
| 105 | $CH_3$ | 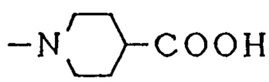 | | Z−Phe − |
| 106 | $CH_3$ | 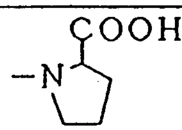 | | Z−Val-Leu − |
| 107 | $CH_3$ | 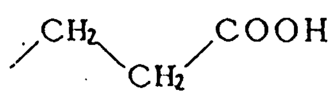 | H | Z−Phe − |
| 108 | $CH_3$ | 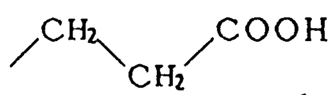 | $CH_3$ | Z−Glu(OBzl)− |
| 109 | $CH_3$ | 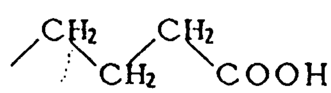 | H | Z−Phe- |

| | | | | |
|---|---|---|---|---|
| 110 | $CH_3$ | 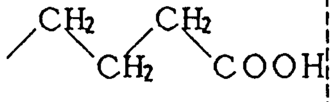 | $CH_3$ | Z—Phe— |
| 111 | $CH_3$ | 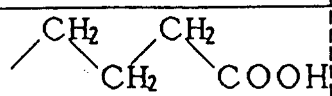 | $nC_3H_7$ | Z—Leu- |
| 112 | $CH_3$ | 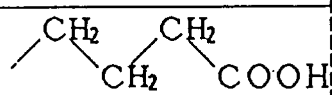 | H | Z—Val-Pro- |
| 113 | $CH_3$ | 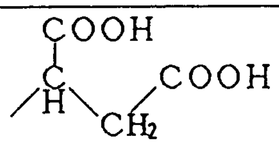 | H | Z—Phe- |
| 114 | $CH_3$ | 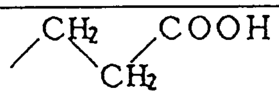 | $CH_3$ | Z—Lys(Z)- |
| 115 | $CH_3$ | 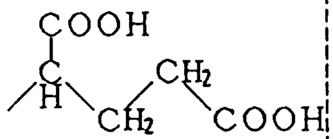 | H | Z—Phe- |
| 116 | $CH_3$ | 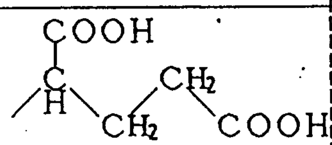 | $CH_3$ | Z—Val- |
| 117 | $CH_3$ | 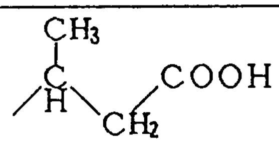 | H | Z—Pro- |

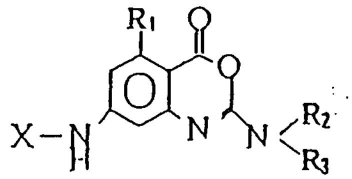

in the case where X is represented with formula [B] $(Y_2-(A_2)_m-A_3-)$

| Compound No. | $R_1$ | $R_2$ | $R_3$ | $Y_2-(A_2)_m-A_3-$ |
|---|---|---|---|---|
| 201 | $CH_3$ | $CH_3$ | H | H-Lys(Z)- (m=0) |
| 202 | $CH_3$ | iPr | H | H-Lys(Z)- (m=0) |
| 203 | $CH_3$ | iPr | H | CPS-Lys - (m=0) |
| 204 | $CH_3$ | iPr | H | H-Glu - (m=0) |
| 205 | $CH_3$ | iPr | H | CPS-Glu - (m=0) |
| 206 | $CH_3$ | $nC_4H_9$ | H | H-Glu (OBzl)- (m=0) |
| 207 | $CH_3$ | $CH_3$ | H | H-Asp - (m=0) |
| 208 | $CH_3$ | iPr | H | H-Asp (OBzl)- (m=0) |
| 209 | $CH_3$ | $CH_3$ | H | H-Leu -Lys - (m=1) |
| 210 | $CH_3$ | $CH_3$ | H | H-Leu -Asp - (m=1) |
| 211 | $CH_3$ | $CH_3$ | H | H-Val -Lys - (m=1) |
| 212 | $CH_3$ | iPr | H | H-Leu -Lys - (m=1) |

| Compound No. | $R_1$ | $R_2$ | $R_3$ | $Y_2-(A_2)_m-A_3-$ |
|---|---|---|---|---|
| 213 | $CH_3$ | iPr | H | H−Gly −Glu − (m=1) |
| 214 | $CH_3$ | iPr | H | CPS−Gly −Glu − (m=1) |
| 215 | $CH_3$ | $CH_3$ | H | H−Gly −Ala −Glu − (m=1) |
| 216 | $CH_3$ | $CH_3$ | H | H−Ala −Asp − (m=1) |
| 217 | $CH_3$ | iPr | H | H−Ala −Glu − (m=1) |
| 218 | $CH_3$ | iPr | H | CPS−Ala −Glu − (m=1) |
| 219 | $CH_3$ | $CH_3$ | H | H−Ala −Lys − (m=1) |
| 220 | $CH_3$ | iPr | H | CPS−Ala −Gly −Lys − (m=1) |

In the above formulas, the abbreviations have following meanings:

| Structural formulas | Abbreviation |
|---|---|
| 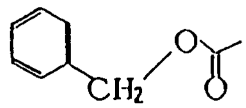 | Z |
| 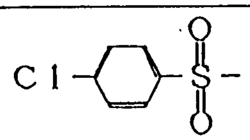 | CPS |
| 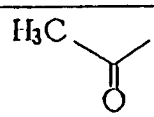 | Ac |
| 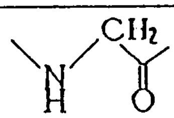 | Gly |
| 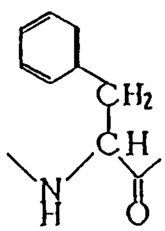 | Phe |
| 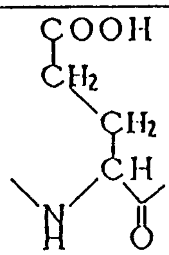 | Glu |
| 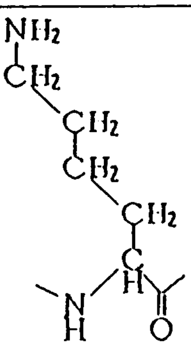 | Lys |

| Structural formulas | Abbreviation |
|---|---|
| 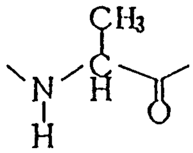 | Ala |
| 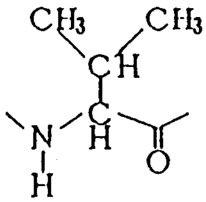 | Val |
| 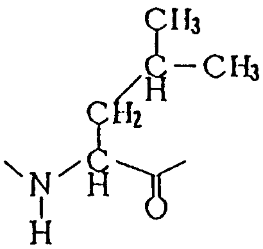 | Leu |
| 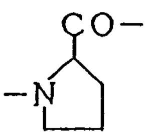 | Pro |
| 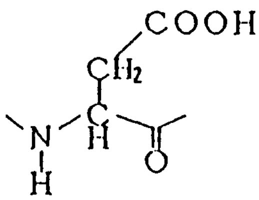 | Asp |

| Structural formulas | Abbreviation |
|---|---|
| 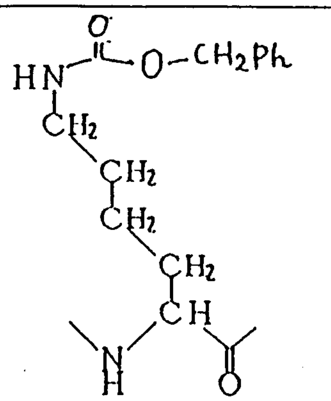 | Lys(Z) |
| 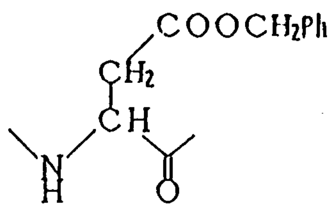 | Asp (OBzl) |
| 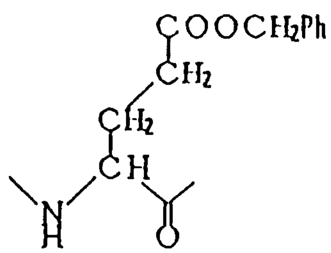 | Glu (OBzl) |

Note: - represents a bond in the structural formulas.

4H-3,1-Benzoxazin-4-one compounds of formula [I] of the present invention shows inhibitory activity against serine protease, particularly stronger inhibitory activity against human leukocyte elastase than other serine proteases, for example, chymotrypsin.

The protease inhibitory activity of the compounds of the present invention is enzymatically determined in vitro by the following method:

Human purulent sputum elastase is regarded as to be identical with human leukocyte elastase (Tomashi et al., J. Biol. Chem., 252, 1917 - 1924 (1977), and commercially available.

Further, methoxysuccinyl-L-alanyl-L-alanyl-L-prolyl-L-valyl-p-nitroanilide (AAPVpNA) has been known as one of synthetic substrates highly selective to human leukocyte elastase (Nakajima et al., J. Biol. Chem., 254, 4027 - 4032 (1979)) and is commercially available. The hydrolysis of AAPVpNA by human purulent sputum elastase can be easily measured by determining the hydrolytically released p-nitroaniline spectrophotometrically. Thus, the hydrolysis of AAPVpNA by human purulent sputum elastase is traced in the absence of or in the presence of a specimen in various concentrations and the concentration of the specimen to inhibit 50% of the hydrolytic reaction ($IC_{50}$) can be decided.

A similar in vitro test can be applied to chymotrypsin, too.

As a chymotrypsin, commercially available α -chymotrypsin of bovine pancreas is used, and the hydrolysis of succinyl-L-alanyl-L-alanyl-L-prolyl-L-phenylalanyl-p-nitroanilide (AAPPpNA), one of synthetic substrates highly selective to chymotrypsin, is traced, as in the above-stated elastase case, in the absence of or in the presence of a specimen in a variety of concentrations to know the concentration of the specimen to inhibit 50% of the hydrolytic reaction ($IC_{50}$).

The intrapulmonary retention of 4H-3,1-benzoxazin-4-one of formula [I] of the present invention after

administered through the respiratory tract can be measured by the following method. A certain dose of a compound of the present invention is given to animals through their respiratory tracts in a solution form and the animals are sacrificed by bleeding at a variety of intervals after the treatment. The compound of the invention remaining in the lungs can be determined by the high performance liquid chromatography of the bronchoalveolar lavage fluids or the pulmonary tissue homogenates.

The efficacy of the compounds of the invention in vivo can be evaluated by the following procedures. In other words, a certain dose of a solution or dispersion of the compound of the invention is given through the respiratory tract, then human purulent sputum elastase is given through the respiratory tract after a certain time. The bronchoalveolar lavage were performed at a definite time after the elastase treatment and the hemoglobin is determined in the bronchoalveolar lavage fluids. The pulmonary hemorrhage is compared with that when a solution containing no compound of the present invention is given to measure the hemorrhage-inhibitory effect of the compounds of the present invention (Bonney et al., J. Cellular Biochemistry, 39, 47 - 53, (1989)).

4H-3,1-Benzoxazin-4-one compounds of the present invention or their salts are used as an active ingredient of pharmaceutical compositions for inhibiting serine proteases.

When they are used as a medicine for inhibiting serine proteases, 4H-3,1-benzoxazin-4-one compounds of formula [I] are in the free form or adduct form with a pharmaceutically permissible acid or base. The acid or base to be used in the salts may be inorganic or organic and there is no limitation, as long as it develops satisfactory effect in the salt form with no or low toxicity to living bodies.

The compounds of general formula [I] or their salts can be mixed with carriers, excipients, solvents, diluents, coloring agents, preservatives, neutralizers, and stabilizers, which are pharmaceutically permissible as active ingredients of pharmaceutical compositions to give desired formulations which are administered perorally, parenterally or through respiratory tracts.

The peroral-dosing medicines may be in the form of a solid formulation such as tablets, granules, powder or capsules or liquid formulation such as syrup, elixir, emulsion or suspension. The parenteral dosing medicine may be in the form of injection solution, suppository, external skin medicine. These medicinal formulations are prepared by a customary process combining a compound of formula [I] or its salt with pharmaceutically permissible adjuvants. Further, sustained release preparations can be produced by known methods.

The solid formulations for peroral administration are produced, for example, by mixing a compound of formula [I] or its salt with an excipient such as lactose, starch, crystalline cellulose, methyl cellulose, glycerol, sodium alginate, arabic gum, calcium hydrogen phosphate, magnesium metasilicate aluminate, calcium lactate, calcium carbonate, sodium chloride, kaolin into a powder, or, when needed, adding a disintegrator such as hydroxypropyl cellulose, polyvinyl pyrrolidone, sucrose, sodium alginate, or sodium hydrogen carbonate into granules. Tablets are prepared by tabletting directly these powder or granules or the combination thereof with a lubricant such as talc or magnesium stearate. In addition, the above-stated granules or tablets are coated with a base such as a methyl methacrylate copolymer or hydroxypropyl methyl cellulose phthalate to give enteric preparations, or coated with ethyl cellulose or hydrogenated oil to form sustained release preparations. Capsule preparations are produced by filling hard capsules with the powder or granules, or by suspending or dissolving the compound of formula [1] or its salt in glycerol, polyethylene glycol or olive oil, then coating with gelatin membranes to form soft capsules.

The liquid formulations for peroral administration are prepared, for example, by dissolving the compound of formula [I] or its salt, a sweetener such as sucrose, glycerol, sorbitol in water, to form a solution or by adding polysolvate 80, sodium carboxymethylcellulose or arabic gum to give an emulsion or suspension.

The injection solution is prepared, for example, by dissolving the compound of formula [I] or its salt in combination with a pH regulator such as sodium monohydrogen phosphate, sodium dihydrogen phosphate, sodium hydroxide, hydrochloric acid, lactic acid, or sodium lactate, an isotonicity such as glucose or sodium chloride and an antioxidant such as ascorbic acid in distilled water for injection, filtering the solution aseptically and charging the filtered solution in ampules, or polyethylene or glass vessels to give single-use injection solutions for subcutaneous, intramuscular, intravenous or arterial purposes or for sustained release injection for a short or prolonged time. The injection formulations of a type for preparing before use are prepared by mixing additionally dextrin, cyclodextrin, mannitol or gelatin, then freeze-drying the mixture under vacuum. The compound of formula [I] or its salt can be encapsuled into liposome or microspheres in accordance with a known method to form injection preparations.

The suppositories can be prepared by melting the compound of formula [I] or its salt together with polyethylene glycol, lanolin, mono-, di- or triglycerides of fatty acids or cacao butter with heat, plasticizing the mixture by cooling, or by suspending or dissolving the active ingredient in soybean oil or polyethylene glycol and coating the solution or suspension with gelatin film or the like.

The external skin preparations are prepared by adding the compound of formula [I] or its salt to polyethylene glycol, white petrolatum or liquid paraffin into ointment, cream, gel or the like.

When the active ingredient is used as an inhalant through respiratory tracts, it is applied in the form of fine particles by a customary inhalation. The fine particles containing the formulation as an active ingredient are in the form of an aerosol or powder and the particle size preferably ranges from 0.5 to 50 $\mu$ m. As an aerosol generator, can be used, for example, a nebulizer of an ultrasonic type or jet type or a spray using lower alkanes or fluorinated alkanes as a propellant. Further, the powder can be dosed with a simplified inhaler interlocked with autogenous or forced respiration.

The concentration of the compound of formula [I] or its salt in the medicinal formulations is not particularly limited, but typically 0.01 to 50 wt.%, preferably 0.1 to 10 wt.% in the formulations.

The dose of these active ingredient is not limited, but suitably 0.1 to 1,000 mg/day/patient, preferably 1 to 200 mg/day/patient. The dosage number is usually 1 to 4 times/day.

The present invention will be illustrated in more detail by the following examples.

Comparative Example

Synthesis of 2-isopropylamino-5-methyl-7-amino-4H-3,1-benzoxazin-4-one

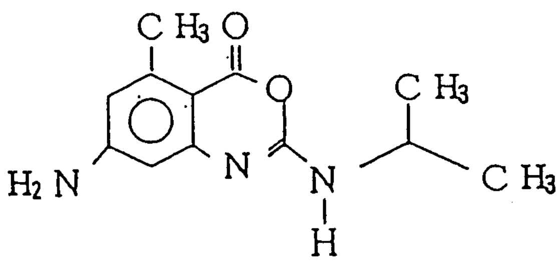

(i) Synthesis of methyl 4-nitro-6-methylanthranilate

4-Methyl-6-nitroanthranyl (40.0 g) was dissolved in methanol (700 ml), then potassium carbonate (4.00 g) was added, and they were refluxed for 3 hours. The methanol was distilled off under reduced pressure, the residue was extracted with water and ethyl acetate. The organic phase was washed with saturated aqueous sodium chloride and dried. After filtration and concentration, the crude product was crystallized form methanol to give the title compound (36.0 g).

NMR spectral data of the compound are given below.

$^1$H-NMR ($CDCl_3$, $\delta$ ppm)

2.94 (3H, s), 3.94 (3H, s), 5.1 - 5.4 (2H, m), and 7.34 (2H, s).

(ii) Synthesis of methyl 2-(3-isopropylureido)-4-nitro-6-methylbenzoate

Methyl 4-nitro-6-methylanthranilate (30.0 g) was dissolved in tetrahydrofuran (300 ml). Acetic acid (75 ml) and isopropyl isocyanate (38 g) were added to the solution and stirred for 19 hours. The crystals precipitated were filtered and dried to give the title compound (31.6 g).

m.p. 208 to 209$^\circ$ C.

NMR spectral data of the compound are given below. $^1$H-NMR ($CDCl_3$, $\delta$ ppm)

1.23 (6H, d, J = 6.4 Hz), 2.53 (3H, s), 3.98 (3H, s), 3.75 - 4.15 (1H, m), 4.3 - 4.55 (1H, m), 7.66 (1H, d, J = 2.0 Hz), 8.55 - 8.7 (1H, m), and 9.03 (1H, d, J = 2.0Hz).

(iii) Synthesis of methyl 2-(3-isopropylureido)-4-amino-6-methylbenzoate

Methyl 2-(3-isopropylureido)-4-nitro-6-methylbenzoate (7.03 g) was dissolved in ethyl acetate (1,000ml). A 10 % palladium-carbon catalyst (1.00 g) was added to the solution and they were stirred at room temperature in a hydrogen atmosphere for 4 hours. After filtration and concentration, the remaining crude product was crystallized from hexane-ethyl acetate to give the title compound (5.58 g).

m.p. 173 to 175$^\circ$ C

NMR spectral data are shown below.

$^1$H-NMR ($CDCl_3$, δ ppm)
1.18 (6H, d, J = 6.4 Hz), 2.38 (3H,s), 3.83 (3H,s), 3.7 - 4.2 (3H, m), 4.4 - 4.65 (1H, m), 6.10 (1H, d, J = 2.4 Hz), 7.65 (1H, d, J = 2.4 Hz), and 9.8 - 10.1 (1H, m).

(iv) Synthesis of 2-isopropylamino-5-methyl-7-amino-4H-3,1-benzoxazin-4-one

Methyl 2-(3-isopropylureido)-4-amino-6-methylbenzoate (9.57 g) was dissolved in conc. sulfuric acid (55 ml) and stirred at room temperature for 2 hours. The reaction mixture was added dropwise in a water-ethyl acetate mixture containing sodium hydrogen carbonate under ice cooling. After dropping, sodium hydrogen carbonate was added for neutralization and the mixture was extracted with ethyl acetate. The organic phase was washed with saturated sodium chloride solution and dried. After filtration and concentration, the residue was crystallized from hexane-ethyl acetate to give the title compound (6.84 g).
m.p. 203 - 205 °C.
NMR spectral data are shown below.
$^1$H-NMR (pyridine-$d_5$, δ ppm)
1.24 (6H, d, J = 6.4 Hz), 2.75 (3H,s), 4.0 - 4.4 (1H, m), 6.2 - 6.6 (3H, brs), 6.65 - 6.8 (1H, brs), and 8.0 - 8.3 (1H, m).

Example 1

Synthesis of 7-(N-benzyloxycarbonyl-L-phenylalanyl)amino-5-methyl-2-(1-carboxyethyl)amino-4H-3,1-benzoxazin-4-one
(Compound No. 101)

(i) Synthesis of 2-benzyloxycarbonyl-3-methyl-5-nitrophenyl isocyanate (the compound of formula (B) in the above-stated route)

Benzyl 4-nitro-6-methylanthranilate (3.55 g) was dissolved in carbon tetrachloride (120 ml). A catalytic amount of triethylamine and bis(trichloromethyl) carbonate (1.23 g) were added to the solution and they were refluxed for 2 hours. Additional 1.23 g of bis(trichloromethyl) carbonate was added and refluxing was repeated for 1 hour. The reaction mixture was cooled down to room temperature, the crystals formed was filtered, the mother liquor was concentrated to collect the title compound (3.51 g).
$^1$H-NMR ($CDCl_3$, δ ppm)
2.38 (3H,s), 5.43 (2H, s), 7.41 (5H, s), 7.7 - 7.9 (2H, m).
IR (neat, $cm^{-1}$)
2280, 1730, 1535, 1350, 1270

(ii) Synthesis of 1-N-(2-benzyloxycarbonyl-3-methyl-5-nitrophenyl)carbamoyl-L-alanine tert-butyl ester (compound C in the above-stated route)

L-alanine tert-butyl ester hydrochloride (606 mg) was suspended in dried THF (10 ml). Triethylamine (371 mg) was added in a nitrogen atmosphere under ice cooling, then they were stirred at 0 °C for 15 minutes and at room temperature for 15 minutes. Then, the solution of 2-benzyloxycarbonyl-3-methyl-5-nitrophenyl isocyanate, which was obtained in (i) (521 mg) in THF (10 ml) was added to the suspension, and they were stirred at room temperature for 14 hours. The reaction mixture was combined with water and extracted with ethyl acetate. The extract was washed with saturated sodium chloride aqueous solution, dried, and concentrated. The residue was purified with a silica gel column to collect the title compound (476 mg).
NMR spectral data are shown below.
$^1$H-NMR ($CDCl_3$, δ ppm)
1.40 (3H, d, J = 7.3 Hz), 1.52 (9H,s), 2.34 (3H, s), 4.25 - 4.65 (1H, m), 5.38 (2H, s), 6.10 (1H, d, J = 7.7 Hz), 7.4 (5H, s), 7.4 - 7.5 (1H, m), 8.77 (1H, brs), 8.91 (1H, d, J = 2.4 Hz).

(iii) Synthesis of N-(5-methyl-7-amino-4H-3,1-benzoxazin-4-on-2-yl)-L-alanine tert-butyl ester (compound (E) in the above-stated route)

1-N-(2-Benzyloxycarbonyl-3-methyl-5-nitrophenyl)carbamoyl-L-alanine tert-butyl ester (3.66 g) was dissolved in ethanol (300 ml), then stirred vigorously together with 10% Pd-C catalyst (810 mg) in a hydrogen

atmosphere at room temperature for 6 hours. The reaction mixture was filtered through a Celite column and the filtrate was concentrated to obtain 1-N-(2-carboxy-3-methyl-5-aminophenyl)carbamoyl-L-alanine tert-butyl ester (2.80 g). The product was dissolved in ethyl acetate (200 ml). This solution was combined with N,N-dicyclohexylcarbodiimide (2.57 g) and they were stirred at room temperature for 15 hours. The crystals formed were separated by filtration, the mother liquor was concentrated and the crude product was purified through a silica gel column to collect the end product (2.20 g).
$^1$H-NMR ($CDCl_3$, $\delta$ ppm)
1.46 (3H, d, J = 7.3 Hz), 1.48 (9H,s), 2.58 (3H, s), 4.0 - 4.5 (4H, m), and 6.25 (2H, s).

(iv) Synthesis of 7-(N-benzyloxycarbonyl-L-phenylalanyl)-amino-5-methyl-2-(1-tert-butoxycarbonylethylamino)-4H-3,1-benzoxazin-4-one (the compound of formula (F) in the above-stated route)

N-Benzyloxycarbonyl-L-phenylalanine (94 mg) and N-methylmorpholine (32 mg) were dissolved in anhydrous THF (5 ml) in a nitrogen atmosphere. After cooling to -18°C, isobutyl chloroformate (43 mg) was added and they were stirred for 2 minutes. N-(5-Methyl-7-amino-4H-3,1-benzoxazin-4-on-2-yl)-L-alanine tert-butyl ester (100 mg) and N-methylmorpholine (32 mg; 1 equivalent) were dissolved in THF (6 ml) and the solution was added dropwise. Stirring was continued one overnight at a temperature ranging -18°C to room temperature, then the reaction mixture was combined with water and extracted with ethyl acetate. The extract was washed with 1 N-hydrochloric acid, saturated aqueous sodium hydrogen carbonate and saturated aqueous sodium chloride, then dried. The crude product was purified through a silica gel column to collect the title compound (101 mg). NMR spectral data are given below.
m.p. 108 to 110°C (from hexane-ethyl acetate)
$^1$H-NMR ($CDCl_3$, $\delta$ ppm)
1.45 (3H, d, J = 7.2 Hz), 1.56 (9H,s), 2.52 (3H, s), 2.95 - 3.2 (2H, m), 4.2 - 5.15 (3H, m), 5.11 (2H, s), 5.80 (1H, brs, d, J = 8.4 Hz), 7.10 - 7.35 (2H, m), 7.17 (5H, s), 7.30 (5H, s), and 8.7 - 8.95 (1H, m).

(v) Synthesis of 7-(N-benzyloxycarbonyl-L-phenylalanyl)-amino-5-methyl-2(1-carboxyethylamino)-4H-3,1-benzoxazin-4-one (the compound of formula (I) in the above-stated route)

7-(N-Benzyloxycarbonyl-L-phenylalanyl)-amino-5-methyl-2-(1-tert-butoxycarbonylethylamino)-4H-3,1-benzoxazin-4-one (188 mg) was dissolved in 4M HCl/dioxane solution (16 ml) and stirred at room temperature for 2 hours. The solution was concentrated, the crude product was purified through a silica gel column to collect the title compound (153 mg).
$^1$H-NMR ($d_6$-acetone, $\delta$ ppm)
1.58 (3H, d, J = 7.3 Hz), 2.59 (3H, s), 2.9 - 3.4 (2H, m), 4.4 - 4.9 (3H, m), 4.9 - 5.4 (1H, m), 5.03 (2H, s) 7.1 - 7.5 (12H, m), and 9.5 - 9.9 (1H, m).

Example 2

Synthesis of 7-(N-benzyloxycarbonyl-L-phenylalanyl)amino-5-methyl-2-(4-carboxypiperidino)-4H-3,1-benzoxazin-4-one
(Compound No. 105)

(i) Synthesis of tert-butyl 1-N-(2-benzyloxycarbonyl-3-methyl-5-nitrophenyl)carbamoyl-4-piperidinecarboxylate
(Compound of formula (C))

Example 1, (ii) was repeated except that tert-butyl 4-piperidinecarboxylate was employed instead of L-alanine tert-butyl ester hydrochloride in example 1, (ii) to produce the title compound. The NMR spectral data of the compound are given below.
$^1$H-NMR ($CDCl_3$, $\delta$ ppm)
1.46 (9H, s), 1.5 - 2.05 (4H, m), 2.2 - 2.5 (1H, m), 2.48 (3H, s), 2.8 - 3.2 (2H, m), 3.75 - 4.1 (2H, m), 5.40 (2H, s), 7.40 (5H, s), 7.63 (1H, brs), 8.97 (1H, brs), and 9.29 (1H, brs).

(ii) Synthesis of tert-butyl N-(5-methyl-7-amino-4H-3,1-benzoxazin-4-one-2-yl)-4-piperidinecarboxylate
(Compound of formula (E))

Example 1, (iii) was repeated except that 1-N-(2-benzyloxycarbonyl-3-methyl-5-nitrophenyl)carbamoyl-L-alanine tert-butyl ester was replaced with the product in (i) in this example. The NMR spectral data of the compound are given below.
$^1$H-NMR ($CDCl_3$, $\delta$ ppm)
1.45 (9H, s), 1.5 - 2.05 (4H, m), 2.2 - 2.5 (1H, m), 2.60 (3H, s), 2.9 - 3.25 (2H, m), 4.05 - 4.45 (4H, m), and 6.23 (2H, s).

(iii) Synthesis of 7-(N-benzyloxycarbonyl-L-phenylalanyl)-amino-5-methyl-2-(4-tert-butoxycarbonyl-piperidino)-4H-3,1-benzoxazin-4-one (Compound of formula (F))

Example 1, (iv) was repeated except that the 2-substituted-5-methyl-7-amino-4H-3,1-benzoxazin-4-one derivative obtained in (ii) was used instead of N-(5-methyl-7-amino-4H-3,1-benzoxazin-4-one-2-yl)-L-alanine tert-butyl ester to produce the title compound. The NMR spectral data of the compound are given below.
$^1$H-NMR ($CDCl_3$, $\delta$ ppm)
1.45 (9H, s), 1.5 - 2.0 (4H, m), 2.2 - 2.6 (1H, m), 2.58 (3H s), 2.9 - 3.25 (4H, m), 4.05 - 4.6 (3H, m), 5.09 (2H, s), 5.4- 5.6 (1H, m), 6.76 (1H, brs), 7.23 (5H, s), 7.30 (5H, s), 7.2 - 7.35 (1H, m), and 8.14 (1H, brs).

(iv) Synthesis of 7-(N-benzyloxycarbonyl-L-phenylalanyl)-amino-5-methyl-2-(4-carboxypiperidino)-4H-3, 1-benzoxazin-4-one (Compound of formula (I))

Example 1, (v) was repeated except that the 7-(N-benzyloxycarbonyl-L-phenylalanyl)amino-5-methyl-2-substituted-4H-3,1-benzoxazin-4-one derivative synthesized in (iii) was employed instead of 7-(N-benzyloxycarbonyl-L-phenylalanyl)amino-5-methyl-2-(1-tert-butoxycarbonylethylamino)-4H-3,1-benzoxazin-4-one to produce the title compound. The NMR spectral data of the compound are given below.
$^1$H-NMR ($d_6$-acetone, $\delta$ ppm)
1.5 - 2.3 (4H, m), 2.58 (3H, s), 2.4 - 2.9 (1H, m), 2.9 - 3.4 (4H, m), 4.1 - 4.7 (3H, m), 5.04 (2H, s), 6.5 - 6.8 (1H, m), 7.00 (1H, brs), 7.26 (5H, s), 7.30 (5H, s), 7.54 (1H, brs), and 9.39 (1H, brs).

Example 3

Synthesis of 7-(N-benzyloxycarbonyl-L-phenylalanyl)amino-5-methyl-2-(N-methyl-3-carboxypropylamino)-4H-3,1-benzoxazin-4-one (Compound No. 110)

(i) Synthesis of tert-butyl 1-N-methyl-1-N-(2-benzyloxycarbonyl-3-methyl-5-nitrophenyl)carbamoyl-4-aminobutyrate
(Compound of formula (C))

Example 1, (ii) was repeated except that tert-butyl 4-methylaminobutyrate was employed instead of L-alanine tert-butyl ester hydrochloride in example 1, (ii) to produce the title compound. The NMR spectral data of the compound are given below.
$^1$H-NMR ($CDCl_3$, $\delta$ ppm)
1.45 (9H, s), 1.6 - 1.95 (2H, m), 2.1 - 2.4 (2H, m), 2.48 (3H, s), 2.96 (3H, s), 3.25 - 3.45 (2H, m), 5.40 (2H, s), 7.40 (5H, s), 7.62 (1H, brs), 9.02 (1H, brs), and 9.20 (1H, brs).

(ii) Synthesis of 2-(N-methyl-3-tert-butoxycarbonylpropylamino)-5-methyl-7-amino-4H-3,1-benzoxazin-4-one - (Compound of formula (E))

Example 1, (iii) was repeated except that 1-N-(2-benzyloxycarbonyl-3-methyl-5-nitrophenyl)carbamoyl-L-alanine tert-butyl ester was replaced with the product in (i) in this example to produce the title compound. The NMR spectral data of the compound are given below.
$^1$H-NMR ($CDCl_3$, $\delta$ ppm)
1.44 (9H, s), 1.65 - 2.05 (2H, m), 2.05 - 2.35 (2H, m), 2.60 (3H, s), 3.09 (3H, s), 3.4 - 3.6 (2H, m), 4.15 (2H, brs), and 6.24 (2H, brs).

(iii) Synthesis of 7-(N-benzyloxycarbonyl-L-phenylalanyl)-amino-5-methyl-2-(N-methyl-3-tert-butoxycarbonylpropylamino)-4H-3,1-benzoxazin-4-one (Compound of formula (F))

Example 1, (iv) was repeated except that the 2-substituted-5-methyl-7-amino-4H-3,1-benzoxazin-4-one derivative obtianed in (ii) was used instead of N-(5-methyl-7-amino-4H-3,1-benzoxazin-4-one-2-yl)-L-alanine

tert-butyl ester to produce the title compound. The NMR spectral data of the compound are given below.
$^1$H-NMR ($CDCl_3$, $\delta$ ppm)
1.44 (9H, s), 1.65 - 2.05 (2H, m), 2.1 - 2.4 (2H, m), 2.59 (3H, s), 3.09 (3H, s), 3.0 - 3.2 (2H, m), 3.35 - 3.65 (2H, m), 4.4 - 4.7 (1H, m), 5.09 (2H, s), 5.35 - 5.55 (1H, m), 6.76 (1H, brs), 7.20 - 7.35 (1H, m), 7.23 (5H, s), 7.30 (5H, s), and 7.95 - 8.05 (1H, brs).

(iv) Synthesis of 7-(N-benzyloxycarbonyl-L-phenylalanyl)-amino-5-methyl-2-(N-methyl-3-carboxypropylamino)-4H-3,1-benzoxazin-4-one (Compound of formula (I))

Example 1, (v) was repeated except that the 7-(N-benzyloxycarbonyl-L-phenylalanyl)amino-5-methyl-2-substituted-4H-3,1-benzoxazin-4-one derivative synthesized in (iii) was employed instead of 7-(N-benzyloxycarbonyl-L-phenylalanyl)-amino-5-methyl-2-(1-tert-butoxycarbonylethylamino)-4H-3,1-benzoxazin-4-one to produce the title compound. The NMR spectral data of the compound are given below.
$^1$H-NMR ($d_6$-acetone, $\delta$ ppm)
1.7 - 2.1 (2H, m), 2.25 - 2.5 (2H, m), 2.59 (3H, s), 2.95 - 3.3 (2H, m), 3.14 (3H, s), 3.5 - 3.8 (2H, m), 4.4 - 4.7 (1H, m), 5.04 (2H, s), 6.5 - 6.7 (1H, m), 7.04 (1H, brs), 7.26 (5H, s), 7.30 (5H, s), 7.52 (1H, brs), and 9.38 (1H, brs).

Example 4

Synthesis of 7-(N-benzyloxycarbonyl-L-phenylalanyl)amino-5-methyl-2-(3-carboxypropylamino)-4H-3,1-benzoxazin-4-one
(Compound No. 109)

(i) Synthesis of tert-butyl 1-N-(2-benzyloxycarbonyl-3-methyl-5-nitrophenyl)carbamoyl-4-aminobutyrate (Compound of formula (C))

Example 1, (ii) was repeated except that tert-butyl 4-aminobutyrate was employed instead of L-alanine tert-butyl ester hydrochloride in example 1, (ii) to produce the title compound. The NMR spectral data of the compound are given below.
$^1$H-NMR ($CDCl_3$, $\delta$ ppm)
1.45 (9H, s), 1.6 - 1.95 (2H, m), 2.1 - 2.5 (2H, m), 2.44 (3H, s), 3.1 - 3.4 (2H, m), 4.8 - 5.05 (1H, m), 5.38 (2H, s), 7.41 (5H, s), 7.61 (1H, brs), 8.50 (1H, brs), and 8.92 (1H, brs).

(ii) Synthesis of 2-(3-tert-butoxycarbonylpropylamino)-5-methyl-7-amino-4H-3,1-benzoxazin-4-one (Compound of formula (E)

Example 1, (iii) was repeated except that 1-N-(2-benzyloxycarbonyl-3-methyl-5-nitrophenyl)carbamoyl-L-alanine tert-butyl ester was replaced with the product in (i) in this example. The NMR spectral data of the compound are given below.
$^1$H-NMR ($CDCl_3$, $\delta$ ppm)
1.45 (9H, s), 1.7 - 2.0 (2H, m), 2.1 - 2.4 (2H, m), 2.61 (3H, s), 3.25 - 3.55 (2H, m), 3.9 - 4.2 (2H m), 4.8 - 5.1 (1H, m), and 6.26 (2H, brs).

(iii) Synthesis of 7-(N-benzyloxycarbonyl-L-phenylalanyl)-amino-5-methyl-2-(3-tert-butoxycarbonylpropyl)-amino-4H-3,1-benzoxazin-4-one (Compound of formula (F))

Example 1, (iv) was repeated except that the 2-substituted-5-methyl-7-amino-4H-3,1-benzoxazin-4-one derivative obtained in (ii) was used instead of N-(5-methyl-7-amino-4H-3,1-benzoxazin-4-one-2-yl)-L-alanine tert-butyl ester to produce the title compound. The NMR spectral data of the compound are given below.
$^1$H-NMR ($CDCl_3$, $\delta$ ppm)
1.44 (9H, s), 1.65 - 2.0 (2H, m), 2.15 - 2.45 (2H, m), 2.62 (3H, s), 3.0 - 3.2 (2H, m), 3.3 - 3.55 (2H, m), 4.05 - 4.25 (1H, m), 4.5 - 4.75 (1H, m), 5.11 (1H, m), 5.4 - 5.7 (1H, m), 6.86 (1H, brs), 7.10 - 7.30 (1H, m), 7.18 (5H, s), 7.31 (5H, s), and 8.2 - 8.6 (1H, m).

(iv) Synthesis of 7-(N-benzyloxycarbonyl-L-phenylalanyl)-amino-5-methyl-2-(3-carboxypropylamino)-4H-3,1-benzoxazin-4-one (Compound of formula (I))

Example 1, (v) was repeated except that the 7-(N-benzyloxycarbonyl-L-phenylalanyl)amino-5-methyl-2-substituted-4H-3,1-benzoxazin-4-one derivative synthesized in (iii) was employed instead of 7-(N-benzyloxycarbonyl-L-phenylalanyl)amino-5-methyl-2-(1-tert-butoxycarbonylethylamino)-4H-3,1-benzoxazin-4-one to produce the title compound. The NMR spectral data of the compound are given below.
$^1$H-NMR ($d_6$-acetone, $\delta$ ppm)
1.7 - 2.1 (2H, m), 2.2 - 2.6 (2H, m), 2.9 - 3.2 (2H, m), 3.25 - 3.5 (2H m), 3.6 - 3.8 (1H, m), 4.5 - 4.85 (1H, m), 5.07 (2H, s), 5.7 - 6.0 (1H, m), 6.90 (1H, brs), 7.10 - 7.30 (1H, m), 7.19 (5H, s), 7.27 (5H, s), and 8.8 - 9.1 (1H, m).

Example 5

Synthesis of 7-(N-benzyloxycarbonyl-L-phenylalanyl)amino-5-methyl-2-(2-carboxethylamino)-4H-3,1-benzoxazin-4-one (Compound No. 107)

(i) Synthesis of 1-N-(2-benzyloxycarbonyl-3-methyl-5-nitrophenyl)carbamoyl-$\beta$ -alanine tert-butyl ester - (Compound of formula (C))

Example 1, (ii) was repeated except that $\beta$ -alanine tert-butyl ester hydrochloride was employed instead of L-alanine tert-butyl ester hydrochloride in example 1, (ii) to produce the title compound. The NMR spectral data of the compound are given below.
$^1$H-NMR ($CDCl_3$, $\delta$ ppm)
1.47 (9H, s), 2.44 (3H, s), 2.4 - 2.7 (2H, m), 3.3 - 3.6 (2H, m), 4.2 - 4.35 (1H, m), 5.38 (2H, s), 7.40 (5H, s), 7.62 (1H, d, J = 0.7 Hz), 8.53 (1H, s), and 8.89 (1H, d, J = 0.7 Hz).

(ii) Synthesis of 2-(2-tert-butoxycarbonylethyl)amino-5-methyl-7-amino-4H-3,1-benzoxazin-4-one (Compound of formula (E))

Example 1, (iii) was repeated except that 1-N-(2-benzyloxycarbonyl-3-methyl-5-nitrophenyl)carbamoyl-L-alanine tert-butyl ester was replaced with the product in (i) in this example. The NMR spectral data of the compound are given below.
$^1$H-NMR ($CDCl_3$, $\delta$ ppm)
1.46 (9H, s), 2.57 (5H, m), 3.62 (4H, m), 4.75 (1H, m), and 6.28 (2H, s).

(iii) Synthesis of 7-(N-benzyloxycarbonyl-L-phenylalanyl)-amino-5-methyl-2-(2-tert-butoxycarbonylethyl)-amino-4H-3,1-benzoxazin-4-one (Compound of formula (F))

Example 1, (iv) was repeated except that the 2-substituted-5-methyl-7-amino-4H-3,1-benzoxazin-4-one derivative obtained in (ii) was used instead of N-(5-methyl-7-amino-4H-3,1-benzoxazin-4-one-2-yl)-L-alanine tert-butyl ester to produce the title compound. The NMR spectral data of the compound are given below.
$^1$H-NMR ($CDCl_3$, $\delta$ ppm)
1.46 (9H, s), 2.45 - 2.65 (2H, m), 2.67 (3H, s), 3.15 (2H, d, J = 6.7 Hz), 3.63 (2H, m), 4.55 (2H, t, J = 6.7 Hz), 5.11 (2H, s), 6.88 (1H, brs), 7.2 - 7.35 (2H, m), 7.27 (5H, m), 7.31 (5H, s), and 8.02 (1H, brs).

(iv) Synthesis of 7-(N-benzyloxycarbonyl-L-phenylalanyl)-amino-5-methyl-2-(2-carboxyethyl)amino-4H-3,1-benzoxazin-4-one (Compound of formula (I))

Example 1, (v) was repeated except that the 7-(N-benzyloxycarbonyl-L-phenylalanyl)amino-5-methyl-2-substituted-4H-3,1-benzoxazin-4-one derivative synthesized in (iii) was employed instead of 7-(N-benzyloxycarbonyl-L-phenylalanyl)amino-5-methyl-2-(1-tert-butoxycarbonylethylamino)-4H-3,1-benzoxazin-4-one to produce the title compound. The NMR spectral data of the compound are given below.
$^1$H-NMR ($d_6$-acetone, $\delta$ ppm)
2.4 - 2.6 (2H, m), 2.65 (3H, s), 3.13 (2H, d, J = 6.7 Hz), 3.69 (2H, m), 4.54 (2H, t, J = 6.7 Hz), 5.12 (2H s), 6.94 (1H, brs), 7.2 - 7.35 (2H, m), 7.27 (5H, m), 7.32 (5H, s), and 9.03 (1H, brs).

Example 6

Synthesis of 7-(N-benzyloxycarbonyl-L-phenylalanyl)amino-5-methyl-2-(1,2-dicarboxyethyl)amino-4H-3,1-be-

nzoxazin-4-one
(Compound No. 113)

(i) Synthesis of 1-N-2-benzyloxycarbonyl-3-methyl-5-nitrophenyl)carbamoyl-L-aspartic acid α , β -di-tert-butyl ester
(Compound of formula (C))

Example 1, (ii) was repeated except that L-aspartatic acid α , β -di-tert-butyl ester hydrochloride was employed instead of L-alanine tert-butyl ester hydrochloride in example 1, (ii) to produce the title compound. The NMR spectral data of the compound are given below.
$^1$H-NMR ($CDCl_3$, δ ppm)
1.46 (9H, s), 1.49 (9H, s), 2.44 (3H, s), 2.75 - 2.9 (2H, m), 4.5 - 4.75 (1H, m), 5.40 (2H, s), 5.7 - 5.9 (1H, m), 7.41 (5H, s), 7.64 (1H, m), 8.72 (1H, brs), and 8.96 (1H, m).

(ii) Synthesis of 2-(1,2-di-tert-butoxycarbonylethyl)amino-5-methyl-7-amino-4H-3,1-benzoxazin-4-one
(Compound of formula (E))

Example 1, (iii) was repeated except that 1-N-(2-benzyloxycarbonyl-3-methyl-5-nitrophenyl)carbamoyl-L-alanine tert-butyl ester was replaced with the product in (i) in this example. The NMR spectral data of the compound are given below.
$^1$H-NMR ($CDCl_3$, δ ppm)
1.44 (9H, s), 1.47 (9H, s), 2.61 (3H, s), 2.90 (2H, d, J = 4.4Hz), 4.12 (2H, brs), 4.6 - 4.8 (1H, m), 5.6 - 5.8 (1H, m), and 6.25 (2H, s).

(iii) Synthesis of 7-(N-benzyloxycarbonyl-L-phenylalanyl)-amino-5-methyl-2-(1,2-di-tert-butoxycarbonylethyl)-amino-4H-3,1-benzoxazin-4-one (Compound of formula (F))

Example 1, (iv) was repeated except that the 2-substituted-5-methyl-7-amino-4H-3,1-benzoxazin-4-one derivative obtained in (ii) was used instead of N-(5-methyl-7-amino-4H-3,1-benzoxazin-4-one-2-yl)-L-alanine tert-butyl ester to produce the title compound. The NMR spectral data of the compound are given below.
$^1$H-NMR ($CDCl_3$, δ ppm)
1.44 (9H, s), 1.49 (9H, s), 2.59 (3H,s), 2.91 (2H, d, J = 4.6 Hz), 3.13 (2H, d, J = 7.0 Hz), 4.4 - 4.8 (2H m), 5.11 (2H, s), 5.35 - 5.55 (1H, m), 5.85 - 6.05 (1H, m), 6.91 (1H, brs), 7.20 - 7.35 (1H, s), 7.23 (5H, s), 7.31 (5H, s), and 8.16 (1H, brs).

(iv) Synthesis of 7-(N-benzyloxycarbonyl-L-phenylalanyl)-amino-5-methyl-2-(1,2-di-carboxyethyl)amino-4H-3,1-benzoxazin-4-one (Compound of formula (I))

Example 1, (v) was repeated except that the 7-(N-benzyloxycarbonyl-L-phenylalanyl)amino-5-methyl-2-substituted-4H-3,1-benzoxazin-4-one derivative synthesized in (iii) was employed instead of 7-(N-benzyloxycarbonyl-L-phenylalanyl)amino-5-methyl-2-(1-tert-butoxycarbonylethylamino)-4H-3,1-benzoxazin-4-one to produce the title compound. The NMR spectral data of the compound are given below.
$^1$H-NMR ($d_6$-acetone, δ ppm)
2.61 (3H, s), 3.05 (2H d, J = 4.6 Hz), 3.21 (2H, d, J = 7.0 Hz), 4.45 - 4.85 (2H, m), 5.11 (2H, s), 5.35 - 5.55 (1H, m), 5.85 - 6.05 (1H, m), 6.91 (1H, brs), 7.2 - 7.35 (1H, m), 7.23 (5H, s), 7.31 (5H, s), and 9.16 (1H, brs).

Example 7

Synthesis of 7-(N-benzyloxycarbonyl-L-phenylalanyl)amino-5-methyl-2-(1,3-dicarboxypropyl)amino-4H-3,1-benzoxazin-4-one
(Compound No. 115)

(i) Synthesis of 1-N-(2-benzyloxycarbonyl-3-methyl-5-nitrophenyl)carbamoyl-L-glutamic acid α , γ -di-tert-butyl ester
(Compound of formula (C))

Example 1, (ii) was repeated except that L-glutamic acid α , γ -di-tert-butyl ester hydrochloride was employed instead of L-alanine tert-butyl ester hydrochloride in example 1, (ii) to produce the title

compound. The NMR spectral data of the compound are given below.
$^1$H-NMR ($CDCl_3$, $\delta$ ppm)
1.45 (9H, s), 1.51 (9H, s), 1.9 - 2.45 (4H, m), 2.42 (3H, s), 4.3 - 4.6 (1H, m), 5.41 (2H, s), 5.5 - 5.7 (1H, m), 7.42 (5H, s), 7.57 (1H, m), 8.77 (1H, brs), and 8.97 (1H, m).

(ii) Synthesis of 2-(1,3-di-tert-butoxycarbonylpropyl)-amino-5-methyl-7-amino-4H-3,1-benzoxazin-4-one (Compound of formula (E))

Example 1, (iii) was repeated except that 1-N-(2-benzyloxycarbonyl-3-methyl-5-nitrophenyl)carbamoyl-L-alanine tert-butyl ester was replaced with the product in (i) in this example. The NMR spectral data of the compound are given below.
$^1$H-NMR ($CDCl_3$, $\delta$ ppm)
1.44 (9H, s), 1.48 (9H, s), 1.8 - 2.4 (4H m), 2.59 (3H, s), 4.23 (2H, brs), 4.3 - 4.6 (1H, m), 5.3 - 5.6 (1H, m), and 6.25 (2H, s).

(iii) Synthesis of 7-(N-benzyloxycarbonyl-L-phenylalanyl)-amino-5-methyl-2-(1,3-di-tert-butoxycarbonyl-propyl)amino-4H-3,1-benzoxazin-4-one (Compound of formula (F))

Example 1, (iv) was repeated except that the 2-substituted-5-methyl-7-amino-4H-3,1-benzoxazin-4-one derivative obtained in (ii) was used instead of N-(5-methyl-7-amino-4H-3,1-benzoxazin-4-one-2-yl)-L-alanine tert-butyl ester to produce the title compound. The NMR spectral data of the compound are given below.
$^1$H-NMR ($CDCl_3$, $\delta$ ppm)
1.44 (9H, s), 1.56 (9H, s), 1.8 - 2.4 (4H m), 2.53 (3H, s), 3.0 - 3.2 (2H, m), 4.25 - 4.8 (2H m), 5.11 (2H, s), 5.6 - 5.8 (1H, m), 6.15 - 6.4 (1H, m), 6.72 (1H, brs), 7.1 - 7.3 (1H, s), 7.19 (5H, s), 7.30 (5H, s), and 8.65 (1H, brs).

(iv) Synthesis of 7(N-benzyloxycarbonyl-L-phenylalanyl)-amino-5-methyl-2-(1,3-dicarboxypropyl)amino-4H-3,1-benzoxazin-4-one (Compound of formula (I))

Example 1, (v) was repeated except that the 7-(N-benzyloxycarbonyl-L-phenylalanyl)amino-5-methyl-2-substituted-4H-3,1-benzoxazin-4-one derivative synthesized in (iii) was employed instead of 7-(N-benzyloxycarbonyl-L-phenylalanyl)amino-5-methyl-2-(1-tert-butoxycarbonylethylamino)-4H-3,1-benzoxazin-4-one to produce the title compound. The NMR spectral data of the compound are given below.
$^1$H-NMR ($d_6$-acetone, $\delta$ ppm)
1.9 - 2.5 (4H, m), 2.59 (3H, s), 3.0 - 3.25 (2H, m), 4.3 - 4.8 (2H, m), 5.17 (2H, s), 5.7 - 5.9 (1H, m), 6.3 - 6.55 (1H, m), 6.82 (1H, brs), 7.1 - 7.3 (1H, s), 7.24 (5H, s), 7.31 (5H s), and 9.32 (1H, brs).

## Example 8

### Synthesis of 7(N-benzyloxycarbonyl-L-alanyl)amino-5-methyl-2-(1-carboxyethylamino)-4H-3,1-benzoxazin-4-one
(Compound No. 102)

(i) Synthesis of 7-(N-benzyloxycarbony1-L-alanyl)amino-5-methyl-2-(1-tert-butoxycarbonylethyl)amino-4H-3,1-benzoxazin-4-one (Compound of formula (F))

Example 1, (iv) was repeated except that N-benzyloxycarbonyl-L-alanine was used instead of N-benzyloxycarbonyl-L-phenylalanine to produce the title compound. The NMR spectral data of the compound are given below.
$^1$H-NMR ($CDCl_3$, $\delta$ ppm)
1.42 (3H, d, J = 7.1 Hz), 1.50 (3H d, J = 6.5 Hz), 1.53 (9H,s), 2.58 (3H, s), 4.2 - 5.1 (3H, m), 5.12 (2H, s), 5.75 (1H, brs), 7.1 - 7.4 (2H, m), 7.32 (5H, s), and 8.8 - 8.95 (1H, m).

(ii) Synthesis of 7-(N-benzyloxycarbonyl-L-alanyl)-amino-5-methyl-2-(1-carboxyethyl)amino-4H-3,1-ben zoxazin-4-one
(Compound of formula (I))

Example 1, (v) was repeated except that the 7-(N-benzyloxycarbonyl-L-alanyl)amino-5-methyl-2-(1-tert-butoxycarbonylethylamino)-4H-3,1-benzoxazin-4-one synthesized in (i) was employed instead of 7-(N-benzyloxycarbonyl-L-phenylalanyl)amino-5-methyl-2-(1-tert-butoxycarbonylethylamino)-4H-3,1-benzoxazin-4-one to produce the title compound. The NMR spectral data of the compound are given below.
$^1$H-NMR ($d_6$-acetone, δ ppm)
1.48 (3H, d, J = 7.1 Hz), 1.51 (3H, d, J = 6.5 Hz), 2.60 (3H s), 4.25 - 5.05 (3H, m), 5.16 (2H, s), 5.5 (1H, brs), 7.1 - 7.35 (2H, m), 7.30 (5H, s), and 9.35 (1H, brs).

Example 9

Synthesis of 7-(N-benzyloxycarbonyl-L-valyl-L-prolyl)amino-5-methyl-2-(3-carboxypropyl)amino-4H-3,1-benzoxazin-4-one
(Compound No. 112)

(i) Synthesis of 7-(N-benzyloxycarbonyl-L-valyl-L-prolyl)-amino-5-methyl-2-(3-tert-butoxycarbonylpropyl)-amino-4H-3,1-benzoxazin-4-one (Compound of formula (F))

Example 1, (iv) was repeated except that the 2-substituted-5-methyl-7-amino-4H-3,1-benzoxazin-4-one derivative obtained in Example 4, (ii) was used instead of N-(5-methyl-7-amino-4H-3,1-benzoxazin-4-one-2-yl)-L-alanine tert-butyl ester, and N-benzyloxycarbonyl-L-valyl-L-proline, instead of N-benzyloxycarbonyl-L-phenylalanine, to produce the title compound. The NMR spectral data of the compound are given below.
$^1$H-NMR ($CDCl_3$, δ ppm)
0.8 - 1.11 (6H, m), 1.44 (9H, s), 1.7 - 2.45 (9H, m), 2.62 (3H, s), 3.35 - 3.9 (4H, m), 4.05 - 4.2 (1H, m), 4.25 - 4.6 (2H, m), 5.09 (2H, s), 5.3 - 5.5 (1H, m), 6.86 (1H, brs), 7.33 (6H, s), and 8.2 - 8.5 (1H, m).

(ii) Synthesis of 7-(N-benzyloxycarbonyl-L-valyl-L-prolyl)-amino-5-methyl-2-(3-carboxypropyl)amino-4H-3,1-benzoxazin-4-one (Compound of formula (I))

Example 1, (v) was repeated except that the 7-(N-benzyloxycarbonyl-L-valyl-L-prolyl)amino-5-methyl-2-(3-tert-butoxycarbonylpropyl)amino-4H-3,1-benzoxazin-4-one derivative synthesized in (i) was employed instead of 7-(N-benzyloxycarbonyl-L-phenylalanyl)amino-5-methyl-2-(1-tert-butoxycarbonylethylamino)-4H-3,1-benzoxazin-4-one to produce the title compound. The NMR spectral data of the compound are given below.
$^1$H-NMR ($d_6$-acetone, δ ppm)
0.8 - 1.11 (6H, m), 1.7 - 2.4 (9H, m), 2.60 (3H, s), 3.4 - 3.9 (4H, m), 4.05 - 4.2 (1H, m), 4.3 - 4.6 (2H, m), 5.11 (2H, s), 5.3 - 5.5 (1H, m), 6.9 (1H, brs), 7.1 - 7.3 (1H, brs), 7.30 (6H, s), and 8.8 (1H, brs).

Example 10

Synthesis of hydrochloride of 7-( ε -N-benzyloxycarbonyl-L-lysyl)amino-5-methyl-2-isopropylamino-4H-3,1-benzoxazin-4-one (Compound No. 202)

(i) Synthesis of 7-( α -N-tert-butoxycarbonyl-ε -N-benzyloxycarbonyl-L-lysyl)-amino-5-methyl-2-isopropylamino-4H-3,1-benzoxazin-4-one

α -N-tert-Butoxycarbonyl- ε -N-benzyloxycarbonyl-L-lysine (732 mg) was dissolved in dried THF (15 ml), and N-methylmorpholine (210 μ l) was added to the solution. The reaction mixture was cooled down to -15 °C and combined with isobutyl chloroformate (981 μ l). Stirring was continued at -15 °C for 5 minutes, then, a solution of 2-isopropylamino-5-methyl-7-amino-4H-3,1-benzoxazin-4-one (300 mg) in dried THF was added dropwise to the mixture. Stirring at -15 °C for about 3 hors was followed by standing gradually up to room temperature and further 12-hour stirring. The reaction mixture was combined with water (100 ml), extracted with ethyl acetate (100 ml) twice, then the collected organic phase was dried over anhydrous magnesium sulfate and the solvent was distilled off. The residue was purified through a silica gel chromatography to give the title compound (813 mg).

The NMR spectral data of the compound are given below.
$^1$H-NMR ($CDCl_3$, δ ppm)
1.27 (6H, d, J = 6.4 Hz), 1.42 (9H, s), 1.4 - 1.8 (6H, m), 2.67 (3H, s), 3.24 (2H, m), 4.00 - 4.42 (2H, m), 4.75 (1H, m), 5.20 (1H, m), 7.10 (1H, m), 7.33 (5H, s), 7.38 (2H, m), and 8.62 (1H, m).

(ii) Synthesis of 7-( ε -N-benzyloxycarbonyl-L-lysyl)amino-5-methyl-2-isopropylamino-4H-3,1-benzoxazin-4-one hydrochloride

7-( α -N-tert-Butoxycarbonyl- ε -N-benzyloxycarbonyl-L-lysyl)amino-5-methyl-2-isopropylamino-4H-3,1-benzoxazin-4-one (650 mg) was dissolved in 4N HCl dioxane (50 ml), stirred at room temperature for 1 hour, and the solvent and hydrochloric acid was distilled off. The residue was combined with benzene, concentrated under reduced pressure to dryness, and the operations were repeated thrice to give the title compound (640 mg).
The NMR spectral data of the compound are given below.
$^1$H-NMR ($D_2O$, δ ppm)
1.32 (6H, d, J = 6.4 Hz), 1.5 - 2.2 (6H, m), 2.77 (3H, s), 3.0 - 3.15 (1H, m), 4.0 - 4.5 (3H m), 5.24 (2H, s), 7.0 - 7.2 (1H, m), 7.40 (5H, brs), and 7.3 - 7.5 (1H, m).

Example 11

Synthesis of hydrochloride of 7-L-glutamylamino-5-methyl-2-isopropylamino-4H-3,1-benzoxazin-4-one - (Compound No. 204)

(i) Synthesis of 7-(N-tert-butoxycarbonyl- γ -tert-butyl-L-glutamyl)amino-5-methyl-2-isopropylamino-4H-3,1-benzoxazin-4-one

Example 10 (i) was repeated except that N-tert-butoxycarbonyl-L-glutamic acid γ -tert-butyl ester was used instead of α-N-tert-butoxycarbonyl-ε-benzyloxycarbonyl-L-lysine to produce the-title compound.

The NMR spectral data of the compound are given below.
$^1$H-NMR ($CDCl_3$, δ ppm)
1.27 (6H d, J = 6.4 Hz), 1.45 (9H, s), 1.47 (9H, s), 1.85 - 2.15 (2H, m), 2.25 - 2.54 (2H, m), 2.68 (3H, s), 3.9 - 4.7 (2H, m), 5.0 - 5.3 (1H, m), 5.5 - 5.6 (1H, m), 7.08 (1H, brs), 7.43 (1H, brs), and 9.03 (1H, m).

(ii) Synthesis of 7-L-glutamylamino-5-methyl-2-isopropylamino-4H-3,1-benzoxazin-4-one hydrochloride

Example 10 (ii) was repeated except that 7-(N-tert-butoxycarbonyl- γ -tert-butyl-L-glutamyl)amino-5-methyl-2-isopropylamino-4H-3,1-benzoxazin-4-one obtained in the above-stated (i) was used instead of 7-( α -N-tert-butoxycarbonyl- ε -N-benzyloxycarbonyl-L-lysyl)amino-5-methyl-2-isopropylamino-4H-3,1-benzoxazin-4-one to give the title compound.

The NMR spectral data of the compound are given below.
$^1$H-NMR ($D_2O$, δ ppm)
1.28 (6H, d, J = 6.4 Hz), 1.9 - 2.2 (2H, m), 2.3 - 2.6 (2H, m), 2.73 (3H, s), 5.3 - 5.8 (1H, m), 7.0 - 7.2 (1H, m), and 7.3 - 7.5 (1H, m).

Example 12

Synthesis of 7-(N-p-chlorobenzenesulfonyl-L-glutamyl)amino-5-methyl-2-isopropylamino-4H-3,1-benzoxazin-4-one
(Compound No. 205)

7-L-Glutamylamino-5-methyl-2-isopropylamino-4H-3,1-benzoxazin-4-one hydrochloride (200 mg) was suspended in methylene chloride (30 ml), hexamethyldisilazane (490 μ l) was added to the suspension and they were stirred at room temperature for 2 hours. p-Chlorobenzeneslfonyl chloride (110 mg) and triethylamine (70 μ l) were added. After stirring at room temperature for 2 hours, triethylamine (140 μ l) was added and the mixture was stirred at room temperature further one overnight. The reaction mixture was poured into 50 ml of 10 % citric acid solution and extracted with ethyl acetate (50 ml) twice. The organic phase was collected, dried over anhydrous magnesium sulfate, filtered, and the solvent was distilled off, The residue was purified through a silica gel chromatography to give the title compound (175 mg).

The NMR spectral data of the compound are given below.
$^1$H-NMR ($d_6$-acetone, δ ppm)
1.29 (6H, d, J = 6.4 Hz), 2.02 (2H, m), 2.44 (2H, t, J = 8.1 Hz), 2.59 (3H, s), 4.09 (1H, m), 6.56 (1H, brs), 6.94 (1H, s), 7.36 (2H, d, J = 6.8 Hz), 7.41 (1H, s), 7.67 (2H, d, J = 6.8 Hz), and 9.38 (1H, s).

Example 13

Synthesis of hydrochloride of 7-(glycyl-L-glutamyl)amino-5-methyl-2-isopropylamino-4H-3,1-benzoxazin-4-one (Compound No. 213)

(i) Synthesis of N-tert-butoxycarbonylglycyl-L-glutamic acid γ -tert-butyl ester

N-tert-Butoxycarbonylglycine (12.4 g) was dissolved in methylene chloride (150 ml) and N,N'-carbonyldiimidazole (11.4 g) was added to the solution. After stirring at room temperature for 15 minutes, glutamic acid γ -tert-butyl α -methyl ester (13.9 g) was added to the mixture and they were stirred one overnight. The reaction mixture was combined with water (100 ml) and 10 % aqueous citric acid to make the pH of the aqueous phase about 2.0 and the phase was separated. The aqueous phase was extracted with ethyl acetate (100 ml), the organic phases were collected, dried over anhydrous magnesium sulfate, filtered, and the solvent was distilled off. The residue was purified through a silica gel chromatography to give N-t-butoxycarbonylglycyl-L-glutamic acid γ -tert-butyl α -methyl ester (21.1 g).

The NMR spectral data of the compound are given below.

$^1$H-NMR ($CDCl_3$, δ ppm)

1.43 (9H, s), 1.47 (9H, s), 1.83 - 2.1 (2H, m), 2.2 - 2.5 (2H, m), 3.75 (3H, s), 3.85 (2H, s), 4.0 - 4.3 (1H, m), 4.6 - 4.8 (1H, m), and 4.95 - 5.20 (1H, m).

The product, N-t-butoxycarbonylglycyl-L-glutamic acid γ -tert-butyl α -methyl ester (21.1 g) was dissolved in methanol (150 ml) and stirred together with 2N aqueous sodium hydroxide (26.9 ml) and water (26.9 ml) at room temperature for 3.5 hours. The methanol was distilled off, water was added (50 ml), and washed with ethyl ether (250 ml). The aqueous phase was separated, then combined with 10 % aqueous succinic acid to adjust the pH to about 3. Additionally the aqueous phase was extracted with ethyl acetate (50 ml) thrice, the organic phases were put together, dried over anhydrous magnesium sulfate, filtered, then the solvent was distilled off to give the title compound (20.1 g).

The NMR spectral data of the compound are given below.

$^1$H-NMR ($CDCl_3$, δ ppm)

1.43 (9H, s), 1.47 (9H, s), 1.8 - 2.1 (2H, m), 2.2 - 2.5 (2H, m), 3.88 (3H, s), 4.0 - 4.35 (1H, m), 4.6 - 4.78 (1H, m), 5.15 - 5.23 (1H, m), and 8.2 - 8.33 (1H, m).

(ii) Synthesis of 7-(N-tert-butoxycarbonylglycyl- γ -tert-butyl-L-glutamyl)amino-5-methyl-2-isopropylamino-4-H-3,1-benzoxazin-4-one

Example 10 (i) was repeated except that N-tert-butoxycarbonylglycyl-L-glutamic acid γ -tert-butyl ester was used instead of α -N-tert-butoxycarbonyl- ε -benzyloxycarbonyl-L-lysine to produce the title compound.

The NMR spectral data of the compound are given below.

$^1$H-NMR ($CD_3OD$, δ ppm)

1.27 (6H, d, J = 6.4 Hz), 1.45 (9H, s), 1.47 (9H, s), 1.80 - 2.17 (2H, m), 2.2 - 2.58 (2H, m), 2.50 (3H, s), 3.88 - 4.10 (3H, m), 4.15 - 4.60 (1H, m), 7.08 (1H, brs), and 7.43 (1H, brs).

(iii) Synthesis of 7-(glycyl-L-glutamyl)amino-5-methyl-2-isopropylamino-4H-3,1-benzoxazin-4-one hydrochloride

Example 10 (ii) was repeated except that 7-(N-tert-butoxycarbonylglycyl- γ -tert-butyl-L-glutamyl)amino-5-methyl-2-isopropylamino-4H-3,1-benzoxazin-4-one obtained in the above-stated (ii) was used instead of 7-( α'-N-tert-butoxycarbonyl- ε -N-benzyloxycarbonyl-L-lysyl)amino-5-methyl-2-isopropylamino-4H-3,1-benzoxazin-4-one to give the title compound.

The NMR spectral data of the compound are given below.

$^1$H-NMR ($D_2O$, δ ppm)

1.30 (6H, d, J = 6.4 Hz), 1.80 - 2.2 (2H, m), 2.25 - 2.6 (2H, m), 2.65 (3H, s), 3.9 - 4.1 (2H, m), 4.2 - 4.5 (2H, m), 7.0 - 7.2 (1H, m), and 7.3 - 7.5 (1H, m).

Example 14

Synthesis of 7-(N-p-chlorobenzenesulfonylglycyl-L-glutamyl)-amino-5-methyl-2-isopropylamino-4H-3,1-benzoxazin-4-one

(Compound No. 214)

Example 12 was repeated except that 7-(glycyl-L-glutamyl)amino-5-methyl-2-isopropylamino-4H-3,1-benzoxazin-4-one hydrochloride was used instead of 7-L-glutamylamino-5-methyl-2-isopropylamino-4H-3,1-benzoxazin-4-one hydrochloride to produce the title compound.

The NMR spectral data of the compound are given below.

$^1$H-NMR ($d_6$-acetone, δ ppm)

1.22 (6H, d, J = 6.3 Hz). 2.02 - 2.20 (2H, m), 2.3 - 2.53 (2H, m), 2.60 (3H, s), 3.77 - 3.82 (2H, m), 3.90 - 4.45 (2H, m), 4.5 - 4.76 (1H, m), 6.90 - 7.05 (1H, m), 6.95 (1H, brs), 7.33 (2H, d, J = 6.8 Hz), 7.48 (1H, brs), 7.70 (2H, d, J = 6.8 Hz), and 9.38 (1H, s).

Example 15

Synthesis of hydrochloride of 7-( β -benzyl-L-aspartylamino)-5-methyl-2-isopropylamino-4H-3,1-benzoxazin-4-one
(Compound No. 208)

(i) Synthesis of 7-(N-tert-butoxycarbonyl- β -benzyl-L-aspartyl)amino-5-methyl-2-isopropylamino-4H-3,1-benzoxazin-4-one

Example 10 (i) was repeated except that N-tert-butoxycarbonyl-L-aspartic acid β -benzyl ester was used instead of α -N-tert-butoxycarbonyl- ε -N-benzyloxycarbonyl-L-lysine to produce the title compound.

The NMR spectral data of the compound are given below.

$^1$H-NMR ($CDCl_3$, δ ppm)

1.26 (6H, d, J = 6.4 Hz), 1.48 (9H, s), 2.67 (3H, s), 2.85 - 3.1 (2H, m), 3.9 - 4.3 (1H, m), 4.5 - 4.9 (2H, m), 5.16 (2H, s), 5.65 - 5.9 (1H, m), 7.05 - 7.15 (1H, m), 7.33 (5H, s), 7.25 - 7.35 (1H, m), and 8.69 (1H, brs).

(ii) Synthesis of 7-(β -benzyl-L-aspartylamino)-5-methyl-2-isopropylamino-4H-3,1-benzoxazin-4-onehydrochloride

Example 10 (ii) was repeated except that 7-(N-tert-butoxycarbonyl- β -benzyl-L-aspartyl)amino-5-methyl-2-isopropylamino-4H-3,1-benzoxazin-4-one obtained in the above-stated (i) was used instead of 7-( α -N-tert-butoxycarbonyl- ε -N-benzyloxycarbonyl-L-lysyl)amino-5-methyl-2-isopropylamino-4H-3,1-benzoxazin-4-one to give the title compound.

The NMR spectral data of the compound are given below.

$^1$H-NMR ($D_2O$, δ ppm)

1.30 (6H, d, J = 6.6 Hz), 2.75 (3H, s), 2.9 - 3.2 (2H, m), 4.3 - 4.9 (2H, m), 5.2 (2H, s), 7.0 - 7.15 (1H, m), 7.25 - 7.35 (1H, m), and 7.40 (5H, brs).

Example 16

Synthesis of hydrochloride of 7-( α -N-p-chlorobenzenesulfo nyl-L-lysyl)amino-5-methyl-2-isopropylamino-4H-3,1-benzoxazin-4-one (Compound No. 203)

(i) Synthesis of α -N-p-chlorobenzenesulfonyl- ε -N-tert-butoxycarbonyl-L-lysine

ε -N-tert-Butoxycarbonyl-L-lysine methyl ester hydrochloride (2.00 g) was suspended in methylene chloride (40 ml) and triethylamine (2.05 g) was added to the solution. Additionally, p-chlorobenzenesulfonyl chloride (1.71 g) was added and they were stirred at room temperature one overnight. The solution was concentrated under reduced pressure, combined with 1N hydrochloric acid, and extracted with ethyl acetate.The organic phase was washed with saturated aqueous sodium hydrogen carbonate and saturated aqueous sodium chloride and dried. The extract was filtered, concentrated and the crude product was purified through a silica gel column to give α -N-p-chlorobenzenesulfonyl- ε -N-tert-butoxycarbonyl-L-lysine methyl ester.

The NMR spectral data of the compound are given below.

$^1$H-NMR ($CDCl_3$, δ ppm)

1.3 - 1.8 (6H, m), 1.44 (9H, m), 2.9 -3.2 (2H, m), 3.52 (3H, s), 3.7 - 4.2 (1H, m), 4.3 - 4.65 (1H, m), 5.1 - 5.35 (1H, m), 7.46 (2H, d, J = 8.8 Hz), and 7.77 (2H, d, J = 8.8 Hz).

The product, α -N-p-chlorobenzenesulfonyl ϵ -N-tert-butoxycarbonyl-L-lysine methyl ester, (2.83 g) was dissolved in methanol (80 ml) and stirred together with 1N aqueous sodium hydroxide (7.4 ml) at room temperature one overnight. The methanol was distilled off, water was added (20 ml), and washed with ethyl ether (50 ml). The aqueous phase was separated, then combined with 10 % aqueous succinic acid to adjust the pH to about 3. Additionally, the aqueous phase was extracted with ethyl acetate (50 ml) thrice, the organic phases were put together, dried over anhydrous magnesium sulfate, filtered, then the solvent was distilled off to give the title compound (2.5 g).

The NMR spectral data of the compound are given below.

$^1$H-NMR ($CDCl_3$, δ ppm)

1.4 - 2.0 (6H, m), 1.43 (9H, m), 2.9 - 3.2 (2H, m), 3.8 - 4.1 (1H, m), 5.0 - 5.5 (1H, m), 5.7 - 6.0 (1H, m), 7.46 (2H, d, J = 8.8 Hz), and 7.77 (2H, d, J = 8.8 Hz).

(ii) Synthesis of 7-(α -N-p-chlorobenzenesulfonyl- ϵ -N-tert-butoxycarbonyl-L-lysyl)amino-5-methyl-2-isopropylamino-4H-3,1-benzoxazin-4-one

Example 10 (i) was repeated except that α -N-p-chlorobenzenesulfonyl-ϵ -N-tert-butoxycarbonyl-L-lysine was used instead of α -N-tert-butoxycarbonyl- ϵ -benzyloxycarbonyl-L-lysine to produce the title compound.

The NMR spectral data of the compound are given below.

$^1$H-NMR ($CDCl_3$, δ ppm)

1.27 (6H, d, J = 6.4 Hz), 1.4 - 2.0 (6H, m), 1.43 (9H, s), 2.67 (3H, s), 2.9 - 3.2 (2H, m), 3.8 - 4.4 (3H, m), 5.1 - 5.5 (1H, m), 5.75 - 6.0 (1H, m), 7.0 - 7.4 (2H, m), 7.48 (2H, d, J = 8.8 Hz), and 7.79 (2H, d, J = 8.8 Hz).

(iii) Synthesis of 7-( α -N-p-chlorobenzenesulfonyl-L-lysyl)amino-5-methyl-2-isopropylamino-4H-3,1-benzoazin-4-one hydrochloride

Example 10 (ii) was repeated except that 7-(α -N-p-chlorobenzenesulfonyl- ϵ -N-tert-butoxycarbonyl-L-lysyl)-amino-5-methyl-2-isopropylamino-4H-3,1-benzoxazin-4-one obtained in the above-stated (ii) was used instead of 7-(α -N-tert-butoxycarbonyl- ϵ -N-benzyloxycarbonyl-L-lysyl)amino-5-methyl-2-isopropylamino-4H-3,1-benzoxazin-4-one to give the title compound.

The NMR spectral data of the compound are given below.

$^1$H-NMR ($D_2O$, δ ppm)

1.30 (6H, d, J = 6.4 Hz), 1.4 - 2.1 (6H, m), 2.70 (3H, s), 3.0 - 3.3 (2H, m), 3.9 - 4.5 (2H, m), 7.0 - 7.4 (2H, m), 7.50 (2H, brs), and 7.80 (2H, brs).

## Example 17

Synthesis of the hydrochloride of 7-(L-alanyl-L-glutamyl)-amino-5-methyl-2-isopropylamino-4H-3,1-benzoxazin- 4-one
(Compound No. 217)

(i) Synthesis of N-tert-butoxycarbonyl-L-alanyl-L-glutamic acid γ -tert-butyl ester

Example 13 (i) was repeated except that N-tert-butoxycarbonylalanine was used instead of N-tert-butoxycarbonyl-glycine to produce the title compound.

The NMR spectral data of the compound are given below.

$^1$H-NMR ($CDCl_3$, δ ppm)

1.40 (3H, d, J = 6.5 Hz), 1.43 (9H, s), 1.47 (9H, s), 1.8 - 2.1 (2H, m), 2.2 - 2.5 (2H, m), 4.0 - 4.4 (2H, m), and 4.7 - 5.0 (2H, m).

(ii) Synthesis of 7-(N-tert-butoxycarbonyl-L-alanyl- γ -tert-butyl-L-glutamyl)amino-5-methyl-2-isopropylamino-4H-3,1-benzoxazin-4-one

Example 10 (i) was repeated except that N-tert-butoxycarbonyl-L-alanyl-L-glutamic acid γ -tert-butyl ester was used instead of α -N-tert-butoxycarbonyl- ϵ -benzyloxycarbonyl-L-lysine to produce the title compound.

The NMR spectral data of the compound are given below.

$^1$H-NMR ($CD_3OD$, δ ppm)

1.27 (6H, d, J = 6.4 Hz), 1.40 (3H, d, J = 6.5 Hz), 1.45 (9H, s), 1.47 (9H, s), 1.8 - 2.17 (2H, m), 2.2 - 2.58

(2H, m), 2.50 (3H, s) 3.8 - 4.5 (3H, m), 7.1 (1H, brs), and 7.43 (1H, brs).

(iii) Synthesis of 7-(L-alanyl-L-glutamyl)amino-5-methyl-2-isopropylamino-4H-3,1-benzoxazin-4-one hydrochloride

Example 10 (ii) was repeated except that 7-(N-tert-butoxy-carbonyl-L-alanyl- γ -N-tert-butyl-L-glutamyl)-amino-5-methyl-2-isopropylamino-4H-3,1-benzoxazin-4-one obtained in the above-stated (ii) was used instead of 7-(α -N-tert-butoxycarbonyl- ϵ -N-benzyloxycarbonyl-L-lysyl)amino- 5-methyl-2-isopropylamino-4H-3,1-benzoxazin-4-one to give the title compound.

The NMR spectral data of the compound are given below.

$^1$H-NMR ($D_2O$, δ ppm)

1.30 (6H, d, J = 6.4 Hz), 1.53 (3H, d, J = 6.5 Hz), 1.8 - 2.17 (2H, m), 2.2 - 2.6 (2H, m), 2.59 (3H, s), 3.9 - 4.6 (3H, m), 7.0 - 7.2 (1H, m), and 7.3 - 7.5 (1H, m).

Example 18

Synthesis of 7-(N-p-chlorobenzenesulfonyl-L-alanyl-L-glutamyl)amino-5-methyl-2-isopropylamino-4H-3,1-benzoxazin-4-one (Compound No. 218)

Example 12 was repeated except that 7-(L-alanyl-L-glutamyl)amino-5-methyl-2-isopropylamino-4H-3,1-benzoxazin-4-one hydrochloride was employed in place of 7-L-glutamylamino-5-methyl-2-isopropylamino-4H-3,1-benzoxazin-4-one hydrochloride to produce the title compound.

The NMR spectral data of the compound are given below.

$^1$H-NMR ($d_6$-acetone, δ ppm)

1.21 (6H, d, J = 6.3 Hz), 1.44 (3H, d, J = 6.2 Hz), 2.0 - 2.25 (2H, m), 2.3 - 2.53 (2H, m), 2.58 (3H, s), 3.90 - 4.45 (3H, m), 4.5 - 4.76 (1H, m), 6.90 - 7.05 (1H, m), 6.9 (1H, brs), 7.36 (2H, d, J = 6.8 Hz), 7.45 (1H, brs), 7.73 (2H, d, J = 6.8 Hz), and 9.1 (1H, s).

Example 19

Protease-inhibitory activity of 4H-3,1-benzoxazin-4-one compounds according to the present invention (Compounds in Examples 1 through 18 and Comparative Examples 1 and 2)

The inhibitory activities of 4H-3,1-benzoxazin-4-one compounds against human purulent sputum elastase and α -chymotrypsin were measured by the method described below. The results are shown in Table 1.

A) Determination of human purulent sputum elastase-inhibitory activity

The buffer solution for determination

The buffer solution containing 0.1 M N-2-hydroxyethylpiperazine-N-2-ethanesulfonic acid, 1 M sodium chloride, 0.1 % polyethylene glycol 6,000. pH: 7.5

Enzyme

Human purulent sputum elastase was obtained from Elastin Product Co. and adjusted to 2 x $10^{-8}$ M with the buffer solution.

Substrate

Methoxysuccinyl-L-alanyl-L-alanyl-L-prolyl-L-valyl-p-nitroanilidewas obtained from Bachem Co. and adjusted to 10 mM with dimethyl sulfoxide.

Procedures

The buffer solution for inhibitory activity determination (2.4 ml) was introduced into the cell which was set to the spectrophotometer (Shimazu UV-2100) equipped with a thermostatic cell holder, and kept at

37°C. The substrate solution (25 μl) and dimethyl sulfoxide containing or not containing an inhibitor were added to the cell and they were stirred. An enzyme solution (50 μl) was added to start the reaction and the hydrolysis of the substrate was measured by change in absorbance at 410 nm wavelength. The inhibitor concentration at which the activity of human purulent sputum elastase was inhibited 50 % ($IC_{50}$) was calculated from the hydrolytic rate of the substrate at the steady state.

B) Determination of α-chymotripsin-inhibitory activity

$IC_{50}$ against α-chymotripsin was determined as in A) determination of human purulent sputum elastase-inhibitory activity except that a solution of bovine pancreas α-chymotripsin (from Sigma Co.) in the buffer solution for activity determination ($2 \times 10^{-9}$ M) was used as an enzyme solution, and succinyl-L-alanyl-L-alanyl-L-prolyl-L-phenylalanyl-p-nitroanilide (from Bachem Co.), as a substrate.

Table 1

| Examples | Compound No. | $IC_{50}$ (M) | | Selectivity |
|---|---|---|---|---|
| | | Elastase | Chymotripsin | |
| 1 | 101 | $5.1 \times 10^{-8}$ | $1.5 \times 10^{-6}$ | 30 |
| 2 | 105 | $9.4 \times 10^{-9}$ | $7.4 \times 10^{-7}$ | 79 |
| 3 | 110 | $2.0 \times 10^{-8}$ | $1.1 \times 10^{-6}$ | 55 |
| 4 | 109 | $5.9 \times 10^{-9}$ | $2.7 \times 10^{-7}$ | 46 |
| 5 | 107 | $6.7 \times 10^{-9}$ | $2.7 \times 10^{-7}$ | 40 |
| 6 | 113 | $4.5 \times 10^{-8}$ | $6.3 \times 10^{-6}$ | 140 |
| 7 | 115 | $5.1 \times 10^{-8}$ | $2.2 \times 10^{-6}$ | 43 |
| 8 | 102 | $5.8 \times 10^{-8}$ | $1.3 \times 10^{-6}$ | 22 |
| 9 | 112 | $8.2 \times 10^{-9}$ | $2.3 \times 10^{-7}$ | 28 |
| 10 | 202* | $1.9 \times 10^{-8}$ | $1.2 \times 10^{-6}$ | 63 |
| 11 | 204* | $2.8 \times 10^{-8}$ | $6.4 \times 10^{-6}$ | 229 |
| 12 | 205 | $2.9 \times 10^{-9}$ | $4.9 \times 10^{-6}$ | 1690 |
| 13 | 213* | $1.4 \times 10^{-8}$ | $3.8 \times 10^{-6}$ | 260 |
| 14 | 214 | $2.0 \times 10^{-9}$ | $1.4 \times 10^{-6}$ | 690 |
| 15 | 208* | $8.2 \times 10^{-9}$ | $5.2 \times 10^{-7}$ | 63 |
| 16 | 203* | $3.1 \times 10^{-9}$ | $1.5 \times 10^{-6}$ | 480 |
| 17 | 217* | $7.6 \times 10^{-9}$ | $1.4 \times 10^{-6}$ | 180 |
| 18 | 218 | $1.7 \times 10^{-9}$ | $1.2 \times 10^{-6}$ | 710 |
| Comparative Exam. 1 | | $1.8 \times 10^{-9}$ | $4.5 \times 10^{-8}$ | 25 |
| Comparative Exam. 2 | | $1.2 \times 10^{-7}$ | $5.2 \times 10^{-7}$ | 4.3 |

Note 1: The compound in comparative example 1 is of formula [I] wherein $R_1$ is $CH_3$;$R_2$, iPr; $R_3$, H; and X is Z-Phe.
Note 2: The compound in comparative example 2 is of formula [I] wherein $R_1$ is $CH_3$;$R_2$, iPr; $R_3$, H; and X is Ac.
Note 3: * means the hydrochloride.

In the table, "selectivity" shows the ratio of $IC_{50}$ against human purulent sputum elastase to $IC_{50}$ against bovine chymotripsin , and the bigger values, the higher selectivity for elastase. The compounds according to the present invention revealed the almost equal level of elastase-inhibitory activity and the equal or superior level of elastase selectivity to those of the compound of comparative example 1, which is one of the compounds disclosed in W088/09790 according to the present inventors. Further, the compounds of the present invention showed higher elastase inhibitory activity and selectivity than those of the compound of comparative example 2 according to Krantz et al., Japanese Patent Laid-open No. S 60-169469 (1985).

<u>Example 20</u>

Intrapulmonary retention of 4H-3,1-benzoxazin-4-one compounds according to the present invention in hamsters, when they are given through respiratory tract

The compounds according to the invention shown in Table 2 were given to Syrian Golden Hamsters (body weight: about 100 g) 200 μ g/kg body weight through respiratory tract to monitor the concentration in their pulmonary tissues with the passage of time. Each compound was dissolved in the concentration of 10 mg/ml of dimethyl sulfoxide and the solution was diluted with the physiological saline solution 50 times before the administration. The 200 μ g/ml solution of a compound was given to hamsters by 1 ml/kg (200 μ g/kg) after the tracheal cannulation under urethane anesthesia.

After a certain time, lungs (wet weight: about 0.5 g) were removed and homogenized in an extraction mixture containing a chloroform/methanol 2 : 1 (v/v) solution (10 ml), physiological saline solution (4.5 ml), acetic acid (0.1 ml) and an internal standard, when the compound can be extracted into the chloroform/methanol 2 : 1 mixture under an acidic condition. The chloroform phase was separated, concentrated under reduced pressure to dryness, and the residue was extracted again with acetonitrile (0.5 ml). The insoluble was removed with 0.45 μ m filter and determined by the reversed phase high performance liquid chromatography. When the extraction was impossible with an organic solvent, bronchoalveolar lavage was performed with physiological saline solution (4 ml), the insoluble was centrifuged off, then, the compound was determined by the reversed phase high performance chromatography. The compounds were detected with their own fluorescence (excitation wavelength: 260 nm, and measurement wavelength: 430 nm). The half life was obtained by plotting the logarithmic values of the compound amount remaining in the pulmonary tissues against time and knowing the inclination of the straight line. The results are given in Table 2 as the half life in the pulmonary tissues. Comparative example 1 is one of the compounds disclosed in W088/09790 and shows that the compounds of the present invention have prolonged half life and greatly improved intrapulmonary retention.

Table 2

| Examples | Compound No. | Intrapulmonary retention ($t_{1/2}$, min) |
|---|---|---|
| 1 | 101 | 90 |
| 2 | 105 | 10 |
| 3 | 110 | 6 |
| 4 | 109 | 15 |
| 5 | 107 | 80 |
| 10 | 202* | 15 |
| 11 | 204* | 26 |
| 12 | 205 | 84 |
| 14 | 214 | 95 |
| 16 | 203* | 90 |
| Comparative example 1 | | < 0.5 |

Note : The compound in comparative example 1 is of formula [I] wherein $R_1$ is $CH_3$;$R_2$, iPr; $R_3$, H; and X is Z-Phe.

* means hydrochloride.

Example 21

Inhibitory effect on pulmonary hemorrhage of of 4H-3,1-benzoxazin-4-one compounds according to the invention in elastase-given hamsters

The compounds according to the invention listed in Table 3 were dissolved or dispersed in 0.01 M phosphate-buffered physiological saline solution (pH 7.5), given to Syrian Golden Hamsters (body weight: about 100 g) 1 mg/kg through respiratory tract, then 30 minutes later, human purulent sputum elastase was

given (1 mg/kg) through respiratory tract. Three hours after elastase administration, the animals were sacrificed by bleeding to open their chests and the bronchoalveolar lavage was performed with physiological saline solution. The hemoglobin in the lavage fluid was determined by the cyanmethemoglobin method.

The results are given in Table 3 as a inhibition rate of pulmonary hemorrhage by the compound according to the invention, compared with 0.01 M phosphate-buffered physiological saline solution (pH 7.5) was given instead of the compounds according to the invention. Comparative example 1 is one of the compounds disclosed in W08/09790 and the inhibitory effect was weak. On the contrary, the compounds according to the present invention revealed excellent inhibitory effect on pulmonary hemorrhage in elastase-given hamsters.

Table 3

| Examples | Compound No. | Pulmonary bleeding inhibition rate (%) |
|---|---|---|
| 1 | 101 | 66.4 |
| 4 | 109 | 81.6 |
| 10 | 202* | 76.2 |
| 11 | 204* | 60.6 |
| 12 | 205 | 71.3 |
| 14 | 214 | 79.5 |
| 16 | 203* | 68.5 |
| Comparative example 1 | | 23.8 |

Note: the compound of comparative example 1 is the same one as in Table 2.

* means hydrochloride.

## Claims

1. 4H-3,1-benzoxazin-4-one compounds of the formula [I]

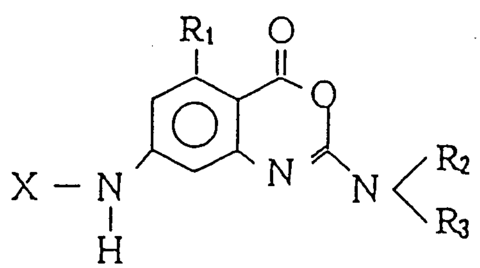

[ I ]

wherein

(i) X is a substituent represented by formula [A]

$Y_1$-$A_1$- [A]

or formula [B]

$Y_2$-$(A_2)$m-$A_3$-      [B]

$A_1$ is an amino acid residue or a peptide which is constituted with 2 or 3 amino acid residues (the side chains of the amino acid residues may be protected),
$A_2$ represents the residue of an amino acid selected from glycine, alanine, valine and leucine or a dipeptide constituted from these amino acids,
$A_3$ represents the residue of an amino acid selected from lysine, glutamic acid and aspartic acid (the side chains of the amino acid residues may be protected),
$Y_1$ represents a protecting group for an amino group,
$Y_2$ represents a hydrogen atom or sulfonyl group (wherein
when the side chain of the amino acid residue in $A_3$ is protected, it represents a hydrogen atom),
m represents 0 or 1;
(ii) $R_1$ represents a hydrogen atom or a lower alkyl;
and
(iii) as to $R_2$ and $R_3$(a) when X represents formula [A]. $R_2$ represents a lower alkyl bearing 1 or 2 carboxyl groups, and $R_3$ represents a hydrogen atom, a lower alkyl, or a lower alkyl bearing 1 or 2 carboxyl groups, or they bond to each other to form a 5, 6 or 7-membered ring substituted with 1 or 2 carboxyl groups or lower alkyl groups bearing 1 or 2 carboxyl groups, or (b) when X represents formula [B], $R_2$ represents a lower alkyl group and $R_3$ is a hydrogen atom;
and their salts.

**2.** The compounds and their salts according to claim 1 wherein X represents the substituent shown by $Y_1$-$A_1$- (formula [A]), $R_1$ is the same as defined in claim 1, and $R_2$ and $R_3$ are the same as defined in the case where X in claim 1 is the substituent represented by said formula [A].

**3.** The compounds and their salts according to claim 2 wherein $R_1$ is a hydrogen atom, a methyl group or an ethyl group.

**4.** The compounds and their salts according to claim 2 wherein $R_2$ is a carboxymethyl, 1-carboxyethyl, 3-carboxypropyl, 1,2-dicarboxyethyl or 1,3-dicarboxypropyl group.

**5.** The compounds and their salts according to claim 2 wherein $R_3$ is a hydrogen atom, a methyl group, an ethyl group or a carboxymethyl group.

**6.** The compounds or their salts according to claim 2 wherein $R_2$ and $R_3$ bond to each other to form 2-carboxypyrrolidine, or 3-carboxypyrrolidine or 4-carboxypiperidine.

**7.** The compounds and their salts according to claim 2 wherein the amino acid residue represented by $A_1$ is selected from the group consisting of alanine, glycine, isoleucine, leucine, phenylalanine, proline, valine, norvaline, norleucine, phenylglycine, lysine whose $\epsilon$ -amino group is protected with a carbobenzoxy group, aspartic acid whose $\beta$ -carboxyl group is protected in the form of a benzyl ester and glutamic acid whose $\gamma$ -carboxyl group is protected in the form of a benzyl ester.

**8.** The compounds and their salts according to claim 2 wherein $Y_1$ is a carbobenzoxy group or methoxysuccinyl group.

**9.** The compounds and their salts according to claim 1 wherein X represents the substituent which is shown by $Y_2$-$(A_2)$m-$A_3$- (formula [B]), $R_1$ is the same as defined in claim 1, and $R_2$ and $R_3$ are the same as defined when X in claim 1 is the substituent represented by said formula [B].

**10.** The compounds and their salts according to claim 9 wherein $R_1$ is a hydrogen atom, a methyl group or an ethyl group.

**11.** The compounds and their salts according to claim 9 wherein $R_2$ is a methyl group, an ethyl group, a propyl group or an isopropyl group.

**12.** The compounds and their salts according to claim 9 wherein $Y_2$ is a hydrogen atom, $A_3$ is a lysine residue whose $\epsilon$ -amino group is protected by a carbobenzoxy group, a glutamic acid residue whose $\gamma$ -carboxyl is protected with a benzyl ester, or an aspartic acid residue whose $\beta$ -carboxyl group is protected with a benzyl ester.

**13.** The compounds and their salts according to claim 9 wherein $Y_2$ is a substituted or unsubstituted aromatic sulfonyl group.

**14.** A pharmaceutical composition comprising a therapeutically effective amount of a compound or its salt according to claim 1 and a pharmaceutically permissible excipient.

**15.** A pharmaceutical composition according to claim 14 which is used for preventing or treating the diseases caused by serine protease.

**16.** A pharmaceutical composition according to claim 15 wherein the serine protease is human leukocyte elastase.

# INTERNATIONAL SEARCH REPORT

International Application No PCT/JP91/00183

**I. CLASSIFICATION OF SUBJECT MATTER** (if several classification symbols apply, indicate all) [6]

According to International Patent Classification (IPC) or to both National Classification and IPC

Int. $Cl^5$ C07D265/24, 413/04, C07K5/06, 5/08, A61K31/535, 37/64

**II. FIELDS SEARCHED**

Minimum Documentation Searched [7]

| Classification System | Classification Symbols |
|---|---|
| IPC | C07D265/24, 413/04, C07K5/06, 5/08, A61K31/535, 37/64 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| X | WO, A, 88-09790 (Teijin Ltd.), December 15, 1988 (15. 12. 88) & EP, A, 317645 | 1-16 |
| A | JP, A, 60-169469 (Syntex (U.S.A.) Inc.), September 2, 1985 (02. 09. 85) & US, A, 4,657,893 | 1-16 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| April 19, 1991 (19. 04. 91) | May 13, 1991 (13. 05. 91) |
| **International Searching Authority** | **Signature of Authorized Officer** |
| Japanese Patent Office | |